(19) Europäisches Patentamt
European Patent Office
Office européen des brevets

(11) EP 0 998 946 A1

(12) **EUROPEAN PATENT APPLICATION**

(43) Date of publication:
**10.05.2000 Bulletin 2000/19**

(51) Int. Cl.[7]: **A61K 49/00**, A61K 47/48

(21) Application number: **99200327.7**

(22) Date of filing: **04.02.1999**

(84) Designated Contracting States:
**AT BE CH CY DE DK ES FI FR GB GR IE IT LI LU MC NL PT SE**
Designated Extension States:
**AL LT LV MK RO SI**

(30) Priority: **14.08.1998 EP 98202735**

(71) Applicant:
**K.U. LEUVEN RESEARCH & DEVELOPMENT**
**3000 Leuven (BE)**

(72) Inventors:
- **Marchal, Guy Jacques Felix**
  **3050 Oud-Heverlee (BE)**
- **Verbruggen, Alfons**
  **3012 Wilsele (BE)**
- **Ni, Yicheng**
  **3020 Herent (BE)**
- **Adriaens, Paul**
  **3020 Winksele (BE)**
- **Cresens, Erwin**
  **3290 Diest (BE)**

(74) Representative: **Duxbury, Stephen**
**Arnold & Siedsma,**
**Sweelinckplein 1**
**2517 GK Den Haag (NL)**

(54) **Non-porphyrin compound for use as a diagnosticum and/or pharmaceutical**

(57) The invention relates to a non porphyrin compound or a pharmaceutically acceptable salt thereof, suitable for in vitro, in vivo and/or ex vivo use as a diagnosticum and/or pharmaceutical, said compound comprising:

- a targeting agent for targeting a specific area, such as an organ and/or tissue,
- a labelling agent L for labelling the targeted area; said targeting agent being connected to the labelling agent L,

wherein said targeting agent comprises one or more substituted and/or unsubstituted organic ring compounds.



Printed by Xerox (UK) Business Services
2.16.7 (HRS)/3.6

## Description

The present invention relates to non-porphyrin compounds or pharmaceutically acceptable salts thereof, suitable for use as drugs for diagnostic and/or therapeutic purpose, in particular for identification and localization of necrotic tissues, to processes for providing such compounds, and to the use of such components for preparing other therapeutic or diagnostic drugs, in particular specific diagnostica for locating necrotic tissues.

Imaging diagnosis has played an indispensable role in medicine because accurate localization and characterization of disease is crucial for therapeutic decision-making and therefore for the overall outcome of patient management. Due to technical innovations magnetic resonance imaging (MRI) as well as other imaging modalities (such as X-ray based radiography, ultrasonography and nuclear scintigraphy) have become much more powerful and versatile. However despite improvements in spatial and temporal resolution, it often remains difficult or even impossible to make an unambiguous diagnosis. This problem can be attributed to the fact that there is substantial overlap in imaging signals between pathological and normal tissues. An approach to solve this problem is to introduce and make use of more specific contrast media or tracers a high discriminatory value, i.e. "specific" agents. Such agents comprise organ, tissue and disease specific contrast agents or tracers which concentrate specifically in targeted organs, tissues or diseases. Tremendous efforts have been made for developing such contrast materials for MRI, however, only a few agents, mainly hepatobiliary, such as Mangafodipir (Mn-DPDP) and Gadobendate dimeglumine (Gd-EOB-DTPA), have shown promise for clinical applications as organ specific contrast agents.

Other tissue or disease selective contrast agents are metalloporphyrins.

The original concept was that some porphyrins exhibit "tumor-targeting" abilities. The assumed accumulation of porphyrins in malignant tumors together with their photosensitizing characteristics lead to their use in porphyrin-mediated cancer photodynamic therapy. By analogy, the so called 'preferential uptake' was utilised for developing paramagnetic metalloporphyrins as 'tumor-seeking' MRI contrast agents.

Recent research has revealed, however, that the apparent specific enhancement of MRI signals in tumors after administration of metalloporphyrins can only be attributed to the presence of non-viable tumor components. Studies in induced 'benign' necrosis further demonstrated that some metalloporphyrins perfectly meet the criteria of diagnostic agents which cause specifically enhanced contrast in necrosis or infarct. However, there are at least two major concerns on the use of porphyrin derivatives, which might prevent the eventual clinical applications of these agents. Porphyrins are characterised with phototoxicity and the same holds true for paramagnetic porphyrins without a central metal chelation such as gadolinium mesoporphyrin or Gadophrin-2. So, the patient has to be kept away from light several days after injection in order to allow a sufficient elimination of the porphyrin from the body. Another associated side-effect is discoloration. Porphyrins are organic dyes with different colors. The subject may temporarily turn green or red after contrast administration due to global distribution and uptake. This may not be accepted by patients, especially for diagnostic purpose.

Therefore, there is an obvious need for searching new agents with similar mechanisms of action or organ/tissue/disease distribution but with minimized toxicity and/or side-effects.

An object of the present invention is to provide alternative contrast agents which aim to solve one or more of these problems. These compounds do not suffer from the drawbacks inherent to the use of porphyrins.

According to a first aspect of the present invention there is provided a compound according to any of the claims 1-20.

These compounds exhibit properties which allow specific localization and identification of necrotic tissues, without the side-effects caused by the porphyrin based agents.

The ability of the compounds to localize and identify necrotic tissues is not based on the fact that they comprise in their structure, e.g., an antibody raised against one or more antigen(s) present in necrotic tissues.

According to a further aspect of the present invention there is provided a compound according to claims 21 and 22.

Results of animal experiments suggested that besides the ability to localize and identify necrotic tissues, other aspecific features of this type of agents can also be exploited. Because of their relatively long plasma half-life they can be used as blood pool agents for MRI or radiographic angiography, especially coronary angiography. Being amphiphylic compounds, both their renal and/or their hepatobiliary elimination pathways allow applications for enhancement of the renal-urinary and hepatobiliary system.

Taking their specific and aspecific targeting properties into account, the compounds according to the present invention can serve as versatile or multipurpose imaging enhancing agents.

By appropriate labeling of the molecules with high density atoms such as iodine or with radioactive atoms, the newly invented compounds can be used in other domains than MRI based imaging diagnosis; these being for example radiography, nuclear medicine.

By labeling with other biochemical or fluorescent indicators, these substances can also be used in macro- and microscopic morphology studies related to tissue death. Another potential of these compounds is to serve as vehicles to carry therapeutic factors such as genes for recovering viability of dead tissues, e.g. in morphologic and functional healing of myocardial and brain infarction.

Further aspects of the invention include the use of the above compounds as necrosis localization agents, for preparing a medicament and/or diagnosticum and to a process for providing these compounds.

The invention will now be further described by way of the following examples and the results of in vitro and in vivo studies, with reference to the accompanying figures wherein:

figure 1 shows the reaction scheme for the bis Gd complex of example 1;
figure 2 shows the Gd content in different organs 24 h after administration of the bis Gd complex of example 1 at 0.05 mmol/kg in rats;
figure 3 shows the Gd content in different organs 10 h after injection of the bis Gd complex of example 1 at 0.05 mmol/kg in a dog;
figure 4 shows the plasma Gd concentration after injection of the bis Gd complex of example 1 at 0.05 mmol/kg in the dog;
figures 5a-d show MR images after administration of the bis Gd complex of example 1, of TTC staining in a rat with reperfused liver infarction at the right lobe;
figure 5e shows a macroscopic photograph of TTC histochemical staining in the same rat with the reperfused liver infarction at the right lobe;
figure 6a-e show MR images of myocardial infarction in the dog; after I.V. administration of the bis Gd complex of example 1;
figure 6f shows a macroscopic picture of TTC histochemical staining of the myocardial infarction in the same dog;
figure 7I-IV show MR images and TTC histochemical staining of a rabbit heart with a small reperfused myocardial infarction, after I.V. administration of the bis Gd complex of example 1;
figure 8 shows nuclear scintigraphy images after administration of $^{99m}$Tc labelled derivative of the compound of example 1 in a rat.

**1. EXAMPLE 1**

**N,N'-Bis(diethylenetriamino penta-acetato)-4,4'-methylene bis(3-hydroxy-2-naphthalene carboxylic hydrazide), Gd-complex, sodium salt (ECIII60)**

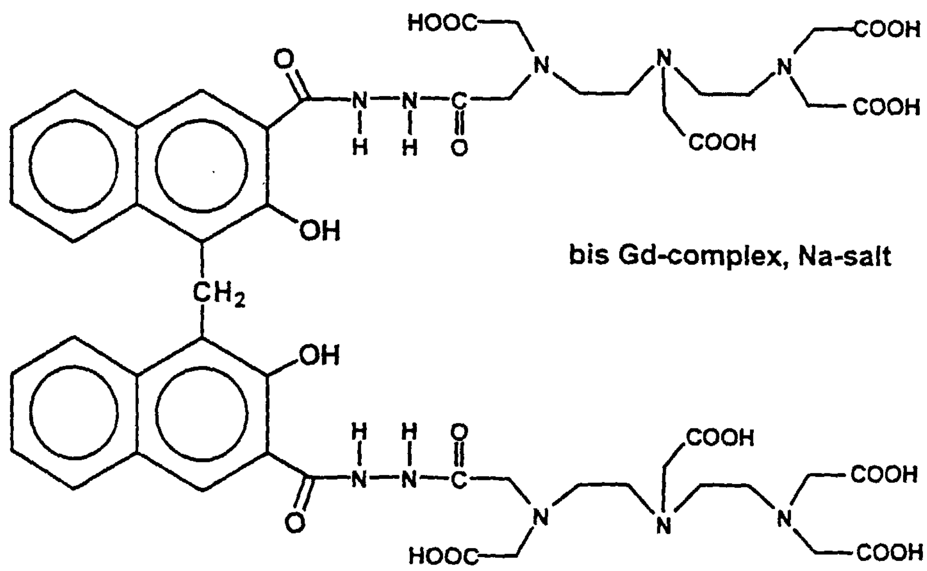

### 1.1 3-Hydroxy-2-naphthalene methyl carboxylate

#### 1.1.1 Methylation with diazomethane

3-Hydroxy-2-naphthalene carboxylic acid (98%, 40g, 208 mmol) was dissolved in ether (1600ml), cooled in ice and methylated with diazomethane. The latter was obtained from 300 mmol of N-methyl-N-nitroso-p-toluluenesulfonamide (Diazald®, Aldrich) according to Hudlicky (1980, J.Org.Chem 45:5377-5378). After methylation the small excess of diazomethane was destroyed with a few drops of acetic acid and the solvent was evaporated in vacuo. The title compound was obtained as a yellow powder.

Yield: (42.93 g (97%. of theory)

#### 1.1.2 Methylation with dimethyl sulfate

The pure crystalline methylester can also be prepared in high yield in a preparative way by the following procedure. 3-Hydroxy-2-naphthalene carboxylic acid (98%, 192 g, 1.0 mol) was dissolved in acetone (2500 ml). Powdered potassium hydrogen carbonate (120 g 1.2 mol) was added followed by distilled water (250 ml). To the stirred solution dimethyl sulfate (111 ml, 1.2 mol) was added dropwise over a period of 4h. The mixture was stirred at room temperature till all starting material was esterified, as judged by TLC (hexane : chloroform, 1:1). Solids present in the reaction mixture were filtered off and washed with acetone. The residue, obtained after removing the solvent in vacuo, was dissolved in dichoromethane and dried with sodium sulfate. Crystallization, necessary to remove some minor fluorescent impurities (TLC), was afforded by reducing the volume and adding methanol to the warm solution. A first crop (164.6 g, 81.4%) was obtained within a few hours. An additional 31.9 g (15.8%) of crystals was recovered after reducing the mother liquor's volume and keeping it overnight at 4°C.

Yield 196.5 g (97%. of theory)
$^{1}$H-NMR ($CDCl_3$)δ

### 1.2 4.4'-Methylene-bis(3-hydroxy-2-naphthalene methyl carboxylate), (pamoic acid dimethylester)

3-Hydroxy-2-naphthalene methyl carboxylate (36.8 g, 187 mmol) was dissolved in acetic acid (280 ml). Sulfuric acid (1.5 ml) and paraformaldehyde (7.50 g, 250 mmol) were added and the mixture was stirred overnight at room temperature. The yellow precipitate was collected by filtration, washed with acetic acid and water and dried in vacuo over phosphorus pentoxide and sodium hydroxide.

Yield 35.41 g (94%. of theory)
$^{1}$H-NMR ($CDCl_3$)δ

### 1.3 4,4'-Methylene-bis(3-hydroxy-2-naphthalene carboxylic hydrazide)

Pamoic acid dimethylester (21 g, 50.5 mmol), pyridine (400 ml), methanol (200 ml) and hydrazine monohydrate (60 ml, 1.24 mol) were refluxed (80°C) for 2h. Solvents and excess hydrazine were removed in vacuo. Traces of pyridine and hydrazine were removed in vacuo azeotropically with distilled water. The residue was suspended in methanol and stirred overnight. The hydrazide was collected by filtration and washed with methanol. Finally the preparation was dried in vacuo over phosphorus pentoxide.

Yield 19.5 g (93%. of theory)
$^{1}$H-NMR (DMSO-d6); δ

### 1.4 N,N'-Bis(diethylenetriamino penta-acetato)-4,4'-methylene bis(3-hydroxy-2-naphthalene carboxylic hydrazide), sodium salt

To a suspension of diethylenetriaminepentaacetic acid ethylester monoanhydride (3.4 g, 8.45 mmol) in dry dimethylformamide (50 ml) and N,N-diisopropylethylamine (3.7 ml, 21 mmol) 1.5 g of the previous dihydrazide (7.2 mmol) was added. The mixture was stirred at room temperature for 5h. Solvent and amine were removed by evaporation in vacuo. The oily residue was dissolved in water; concentrated sodium hydroxide was added to reach a pH>12. After all ethyl ester groups were hydrolysed, as judged by HPLC, water was evaporated in vacuo at 60°C till nearly dry. The residue was diluted with water and the pH adjusted to 7.4 with HCl. The mixture, that contained 76% of the diDTPA-derivative (HPLC) was fractionated by reversed phase chromatography on silica C-18 using a water-acetonitrile gradient. Fractions containing pure compound were pooled and the solvent was removed in vacuo till almost dry. Methanol was added to induce precipitation. The precipitate was collected by filtration and dried in vacuo over phosphorus pentoxide.

Yield 4.33 g (46%. of theory)
$^1$H-NMR (DMSO-d6/$D_2O$); δ

**1.5 N,N'-Bis(diethylenetriamino penta-acetato)-4,4'-methylene bis(3-hydroxy-2-naphthalene carboxylic hydrazide), Gd-complex, sodium salt**

The DTPA-derivative (1.5 g, 1.15 mmol) was desolved in water (90 ml) and titrated with a stock solution of gadolinium(III) acetate hydrate in water (0.24 M). During the addition of Gd the pH was maintained at 7.4 with sodium hydroxide (1.0 M). Progress in complexation was monitored by HPLC. For desalting the preparation was applied on a C18-silicagel column that was rinsed with distilled water. The Gd-compound was eluted with 30% acetonitrile. Solvents were removed in vacuo.

Yield 1.6 g (92%. of theory)
Mass; calculated for $C_{51}H_{54}Gd_2Na_2N_{10}O_{22}$ (Gd=157) 1519.5; m/e found

**2. EXAMPLE 2**

**N,N'-Bis (diethylenetriamino penta-acetato)-4,4'-methylene bis(1-hydroxy-2-naphthalene carboxylic hydrazide), Gd-complex, sodium salt**

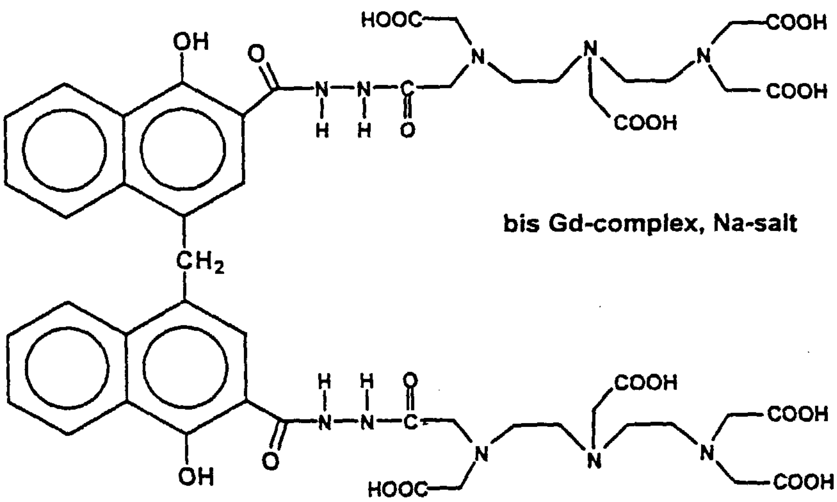

bis Gd-complex, Na-salt

**2.1 4,4'-Methylene-bis(1-hydroxy-2-naphthalene methyl carboxylate)**

1-Hydroxy-2-naphthalene carboxylic acid (37.60 g, 200 mmol) was dissolved in ether (1200 ml) and after cooling in ice was methylated with diazomethane from 300 mmol of Diazald®. The solution was evaporated in vacuo; the solid residue (pure by TLC on silicagel; hexane-chloroform 1:1) was dissolved in acetic acid (300 ml) which contained sulfuric acid (1.5 ml) and paraformaldehyde (7.50 g, 250 mmol) and stirred overnight at room temperature. The thick suspension was filtered and the precipitate was washed with acetic acid and water and dried in vacuo over phosphorus pentoxide and sodium hydroxide.

Yield 40.38 g. (97%. of theory)

$^1H$-NMR ($CDCl_3$)δ 3.85 (s, 6H, 2 carboxymethyls), 6.40 (s, 2H, methylene), 7.25, 7.37 (2s, 4H, aromatic hydrogens), 7.58, 7.61, 8.5 (3 m, 6H, aromatic hydrogens) 11.95 (s, 2 phenols).

**2.2 4,4'-Methylene-bis (1,-hydroxy-2-naphthalene carboxylic hydrazide)**

The dimethyl ester (13.31 g, 32 mmol) was dissolved in a mixture of pyridine (300 ml), methanol (100 ml) and hydrazine monohydrate (41 ml, 0.85 mol) and refluxed 4 h. After standing overnight at 2°C, 8.53 g (64%) of a precipitate (washed with cold methanol) was obtained. Concentrating the mother liquor yielded a second crop of 4.36 g (33%).

Yield 12.89 g. (97%. of theory)

**2.3 N,N'-Bis(diethylenetriamino penta-acetato, monoethylester)-4,4'-methylene bis(1-hydroxy-2-naphthalene carboxylic hydrazide), sodium salt**

The dihydrazide (4.16 g, 10 mmol) was suspended in dry dimethylformamide (200 ml) and N,N-diisopropylethylamine (18 ml, 100 mmol); after addition of DTPA-mono anhydride mono ethyl ester (9.07 g, 22.5 mmol), the mixture was stirred overnight at room temperature. Solvent and amine were removed by evaporation in vacuo and the oily residue was dissolved in 1 M sodium bicarbonate (100 ml) to give a pH of 7.0. This solution was purified on a C18-silicagel column using a gradient of acetonitrile in a sodium phosphate-EDTA buffer of pH 7.0. Fractions containing the pure compound were pooled, evaporated to dryness, redissolved in water and desalted on a C18 silicagel column in water; the product was washed from the column with 25% acetonitrile.

Yield 3.0 g. (24%. of theory)
$^1H$-NMR (DMSO-d6); δ 1.18 (t,6H,2x $CH_2\underline{CH_3}$), 2.93 → 3.54 (m, 36 H, methylenes of DTPA), 4.04 (t, 4H, 2x $\underline{CH_2}CH_3$), 4.59 (s, 2H, methylene), 7.5, (m, 6H), 8.0 (d, 2H) and 8.3 (d,2H) (all aromatic hydrogens).

**2.4 N,N'-Bis(diethylenetriamino penta-acetato)-4,4'-methylene bis(1-hydroxy-2-naphthalene carboxylic hydrazide), Gd-complex, sodium salt**

The hydrazide-DTPA-ethyl ester (1.45 g, 1.18 mmol) was first hydrolysed with 5 N sodium hydroxide at pH 13 (monitored by HPLC). After 2 hours the solution was neutralized and gadolinium triacetate dihydrate (0.88 g, 2.375 mmol) was added. The solution was desalted on a C18-silicagel column in water; the Gd-complex was eluted with 30% acetonitrile.

Yield 1.42 g = 80%
Mass; calculated for $C_{51}H_{54}Gd_2Na_2N_{10}O_{22}$ (Gd = 157) 1519.5; m/e found

## 3. EXAMPLE 3

**N,N'-Bis(diethylenetriamino penta-acetato)-4,4'-methylene bis(3-hydroxy-2-naphthalene carboxylic hydrazide), technetium-99m complex, sodium salt**

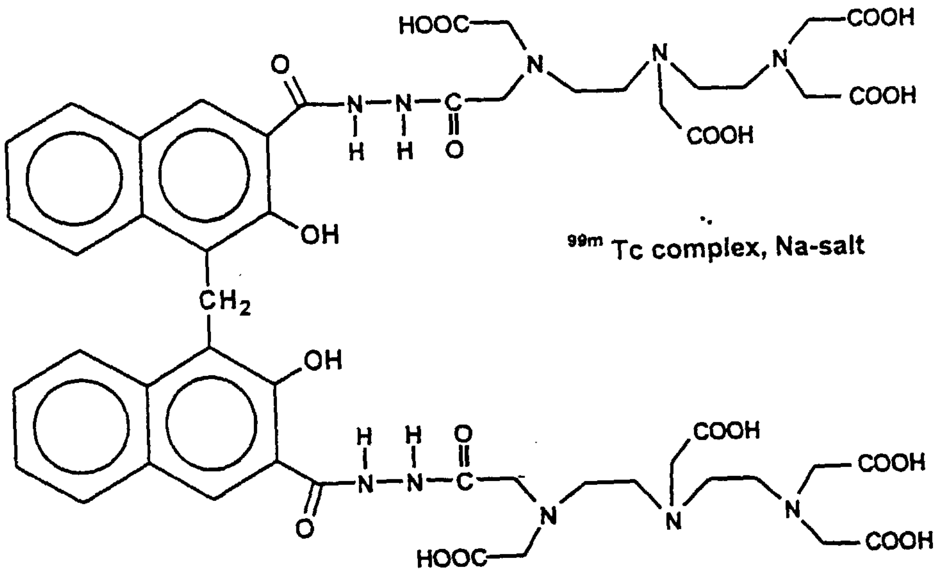

To a solution of 5 mg of N,N'-Bis(diethylenetriamino penta-acetato)-4,4'-methylene bis(3-hydroxy-2-naphthalene carboxylic hydrazide (see example 1.4) in 1 ml water at pH 5.0 in a closed 10 ml vial were added 0.25 mg $SnCl_2.2H_2O$ dissolved in 25 μl 0.05 N hydrochloric acid and 740 MBq technetium-99m in the form of sodium pertechnetate, dissolved in 1 ml normal saline. The mixture was incubated at room temperature for 10 min and analyzed by reversed phase HPLC (silica-C18) using a phosphate buffer (pH 7.0) acetonitrile gradient. The technetium-99m complex eluted as a single peak.

**In vivo assessment of contrast enhancing properties**

Animal Models:

Two effective and reproducible animal models have been induced and used in the in vivo assessment of newly synthesized agents.

1. Reperfused hepatic infarction in rats: adult Wistar rats weighing about 350 grams were anesthetized with intraperitoneal injection of pentobarbital (Nembutal®, 40 mg/kg, Sanofi Sante Animale, B-1130 Brussels, Belgium). Under laparotomy, reperfused hepatic infarction was induced by temporarily clamping the hilum of the right liver lobes for 3 hours. The abdominal cavity was then closed and the rats were allowed to recover from surgery.
2. Acute myocardial infarction in rabbits and dogs: under general anesthesia with Nembutal® at 6 mg/kg/h and intubated ventilation, adult New Zealand rabbits of 3 kg or Mongrel dogs of 20 kg underwent open-chest surgery through the seventh intercostal space. The left anterior descending (LAD) or circumflex (Cx) branch of the coronary artery was encircled and occluded with a snare-occluder for 150 minutes and reopened by losing the occluder to induce irreversible reperfused AMI. The thoracic cavity was closed by layers with the free air expelled.

Agents and dosage:

The synthesized agents according to examples 1-3 were evaluated at a standard intravenous dose of 0.05 mmol/kg (based on metal content) according to the previous experiences with metalloporphyrins. For inter- and intra-individual comparisons, one known necrosis-avid porphyrin agent Gadophrin-2 (gadolinium mesoporphyrin or Gd-MP) at 0.05 mmol/kg and one commercially available MRI contrast agent Gd-DTPA (Magnevist®) at 0.1 mmol/kg were used

as specific and nonspecific control agents respectively.

MR Imaging and contrast enhancement:

The above-mentioned anesthetic regimes were also used during MR imaging. The tail or ear vein of the rat and rabbit or one peripheral vein of the dog was cannulated with an infusion set connecting to a syringe loaded with a contrast agent. The animal was introduced in an appropriate coil and imaged with a 1.5 T Magnetom Vision scanner (Siemens, Erlangen, Germany). For MR imaging in rats, T1 ( TR/TE = 420 ms/12 ms ) and T2 ( TR/TE = 3000 ms / 90 ms ) spin echo sequences were applied with slice thickness of 2 mm (without gap), the field of view 7.5 x 10 cm, the matrix of 192 x 256 and two averaged acquisitions, resulting in about 3 minutes of measurement. A glass tube containing 0.02% $CuSO_4$ solution was placed aside the rat as external standard for normalization of signal intensity (SI) values. Postcontrast MRI was taken every 10 minutes for the first hour (early phase) and up to 24 to 48 hours (late phase). For MR imaging in rabbits and dogs, in vivo ECG gated T1 weighted turbo FLASH and T2 weighted HASTE MRI, and T1 and T2 weighted spin echo MRI of excised heart were performed before and serially after iv injection of the agent.

All MR images were reviewed qualitatively and analyzed quantitatively. The SI of necroses (i.e. hepatic and myocardial infarcts) as well as normal liver or myocardium, kidney, muscle and the external standard tube were measured using a monitor-defined circular region of interest (ROI). In the same animal, the size and location of the ROI were kept constant for measurement at different time points. Each lesion was measured on 1 to 3 slice(s) depending on lesion size. The obtained SI values were normalized using the formula: $SI_{normalized} = SI_{poet} \times (SI_{standard\ pre}/SI_{standard\ post})$ . The conspicuity of the necrotic lesions or infarcts was expressed as contrast ratio (CR) between necrosis and adjacent normal tissue and was calculated as $CR = SI_{necrosis}/SI_{normal}$ at certain time point. In cardiac study, findings of MRI were planimetrically correlated with results of triphenyltetrazolium chloride (TTC) staining. Statistical analyses were done by using a computer program.

Results: Before contrast administration, the necroses or hepatic and myocardial infarcts were undetectable with T1 weighted MRI. Unlike Gd-DTPA, which only enhances nonspecifically the lesions at the early phase (<2h), the compounds according to examples 1-3 induced strong and persistent contrast enhancement of the lesions with the degree and duration equal to that with Gadophrin-2, as illustrated in Example 4 to 6, below.

**Scintigrafic imaging and infarction to normal tissue radioactivity ratio**

The compound, described in example 3, was injected in a dose of 74 MBq in a rat with reperfused hepatic infarction. This animal was prepared as described above. Scintigraphic images were acquired during 1 to 5 minutes at regular time intervals up to 24 h after injection, using a Triad-gamma camera equipped with an ultra-high resolution parallel collimator. Clear visualization of the infarcted region was obtained on the images acquired 20 h after injection.

After sacrificing the animal, organs were isolated and necrotic and viable liver parts were separated and weighed. Their 99m Tc radioactivity was measured using a sodium iodine scintillation detector coupled to a multichannel analyzer. The activity per gram tissue was a factor of 3.2 higher in necrotic liver tissue as compared to normal liver tissue.

**Biodistribution data:**

Methods: Biodistribution of compound ECIII60 according to example 1, was studied by measuring MRI signals intensities in function of time (dynamic: pre and up to 48h post) and by sampling tissues of multiple organs at 24 h after contrast for Gd concentration measurement (static at 24 h) with inductively coupled plasma atomic emission spectrometry (ICP-AES) techniques.

Results and remarks: Gadolinium content in necrotic liver is more than 3 times the value in normal liver (Fig. 2), while about a one-to-one proportion was found with Gadophrin-2, suggesting a more favorable necrosis affinity. The relative large amount of Gd found in normal kidney and liver is mainly due to hepatobiliary and renal pathways of the agent. More than 25 times higher concentration was found in infarcted than in normal myocardium (Fig. 3). The plasma concentration of the agent in function of time was also obtained from the animals (Fig. 4).

Toxicity profile

General condition of the rats, rabbits and dogs after intravenous dose at 0.05 mmol/kg was stable with no

mortality and visible side effects related to the use of the agents. No phototoxicity and discoloration were observed when animals were exposed to daylight after administration of the contrast agent. The acute lethal toxicity (LD50) obtained with ECIII-60 in mice and rats is about 3.8 mmol/kg (see in table 1), which is comparable with that of Magnevist (Gd-DTPA) if the effective doses are taken into account. The animals which had tolerated a large IV dose during LD50 tests survived normally for more than one year with an average body weight similar to that in those animals of the same age but without contrast injection.

Table 1

| Acute Lethal Toxicity ($LD_{50}$) with ECIII-60 | | | |
|---|---|---|---|
| Animals | Dose | No. Deaths/No. animals | Interpolated LD50 (mmol/kg) |
| mice | 2.15 mmol/kg | 0/4 | |
| | | | |
| rats | 2.5 mmol/kg | 0/4 | |
| | | | |
| rats | 5.0 mmol/kg | 4/4 | |
| | | | |
| mice | 5.0 mmol/kg | 3/3 | (2.5 +5.0)/2 =3.8 mmol/kg0 |

**Hepatic and renal toxicity studies:**

Animals: 4 adult New-Zealand rabbits.
Dose of contrast agent: ECIII60 iv at 0.05 mmol/kg (≥ recommended dose)

**Table 2** basic serologic parameters of hepatic and renal functions

| Items | Alb (g/L) | | | ALT (U/L) | | | ALP (U/L) | | | TB (mg%) | | | Urea (mg%) | | | Cr (mg%) | | |
|---|---|---|---|---|---|---|---|---|---|---|---|---|---|---|---|---|---|---|
| Phases* | pre | early | late | pre | early | late | pre | early | late | pre | early | late | pre | early | late | pre | early | late |
| Rabbit-1 | 33 | 32 | 29.8 | 59 | 60 | 56 | 238 | 235 | 215 | <.10 | .10 | <.10 | 53 | 55 | 33 | 1.29 | 1.14 | 1.11 |
| Rabbit-2 | 29.5 | 29.2 | 28.3 | 45 | 45 | 46 | 127 | 127 | 122 | <.10 | <.10 | .11 | 47 | 46 | 33 | 1.22 | 1.16 | 1.08 |
| Rabbit-3 | 32.1 | 30.8 | 29.9 | 71 | 68 | 72 | 178 | 169 | 178 | <.10 | .10 | .11 | 69 | 70 | 34 | 1.12 | 0.95 | 0.94 |
| Rabbit-4 | 32.3 | 31.8 | 29.9 | 91 | 92 | 73 | 240 | 238 | 217 | <.10 | .10 | .13 | 57 | 59 | 38 | 1.39 | 1.29 | 1.13 |

Note: *pre: pre-injection; early: 2h post-injection; late: 24h post-injection.
Alb: albumin; ALT: alanine aminotransferase; ALP: alkaline phosphatase; TB: total bilirubin; Cr: creatinine.

## 4. EXAMPLE 4

### (MRI enhancement of liver infarction):

MR images and macroscopic photograph of TTC histochemical staining in a rat with reperfused liver infarction at the right lobe, were obtained:

a-d. Transverse T1 weighted spin echo images before (a), 5 min (b), 60 min (c), and 24 h (d) after injection of ECIII-60 (0.05 mmol/kg). Before injection, the infarcted liver lobe was undistinguishable from other normal lobes (a). Shortly after injection, while other lobes were strongly enhanced, the infarcted lobe became slightly hypointense except those lobar vessels which were perfused by the blood and porphyrin agent (b). During the first postcontrast hour, the necrosis-to-normal contrast was gradually reversing, i.e. while decreasing in normal liver, the signal intensity in infarcted lobe was increasing (c). At 60 minutes after injection, the signal intensity of infarcted liver already surpassed that of normal liver though not yet homogeneous (c). The enhancement of the necrosis became homogeneous within a couple of hours and the contrast reached maximum at 24 hours (d) (see Fig. 5).

e. Macroscopic photograph of TTC stained liver section displays the infarcted (pale) and normal (brick red in colored photographs) liver lobes. Within the infarcted right lobe, a few areas of viable tissue exist underneath the capsule and alongside intrahepatic vessels, which correspond well with the MR images (see Fig. 5).

## 5. EXAMPLE 5

### (MRI enhancement of myocardial infarction in the dog):

ECG triggered, T1-w segmented turbo-FLASH in vivo MR images, T1- and T2-w spin echo ex vivo Mr images, and macroscopic picture of TTC histochemical staining of a dog with an acute reperfused myocardial infarction at the posterior papillary muscle supplied by left circumflex coronary artery which was temporarily obstructed for 150 min. Before contrast, the infarct is invisible (a). Ten minutes after IV injection of ECIII-60 at 0.05 mmol/kg, the signals of not only the blood pool at the right (R) and left (L) ventricles but also the papillary muscle (arrow) are strongly enhanced (b). Eighteen hours after contrast, a "light bulb" sign due to the extremely strong enhancement is shown at the infarcted papillary muscle (c). Ex vivo T1-w MRI (d) also demonstrates this "light bulb" sign, which on the contrary appears hypointense on T2-w MRI (e) as a result of T2 shortening effect with highly concentrated agent accumulated in this necrotic papillary muscle. This finding is consistent with that of Gd tissue content measurement (Fig. 3). The pale area on the corresponding TCC stained heart section (f) matches perfectly with the hyperintense infarct as on MR images (c and d). The dark areas in the infarct correspond to intralesional hemorrhage (see Fig. 6).

## 6. EXAMPLE 6

### (MRI enhancement of myocardial infarction in the rabbit)

T1-w spin echo MR images and macroscopic pictures of TTC histochemical staining of a rabbit heart with a small reperfused myocardial infarction at the posterior wall supplied by left circumflex coronary artery (Cmx) which was temporarily obstructed for 3h. Eight serial short axis MR images from the apex to the base of the heart were taken immediately after sacrifice but 18 hours after IV injection of ECIII-60 at 0.05 mmol/kg (I a-h). The persistently enhanced small but transmural infarct is only visible on 2 slices (e and f, arrows). R and L represent the right and left ventricles. The corresponding serial TTC stained sections (II) as well as infarct-containing "zoom-in" MR images (III) and TTC stained pictures (IV) are shown (see Fig. 7).

## 7. EXAMPLE 7

Nuclear scintigraphy 24 hours after administration of the title compound of example 3, at 2 µci/rat, wherein K: kidney; Control: normal control rat; Necrotic: rat with reperfused right liver lobe infarction; Arrow indicates the hot spot caused by accumulation tracer in the necrotic liver lobe only. This imaging result has been confirmed by radioactivity counting with a factor of more than 3 between infarcted and normal liver tissues.

The invention is not limited to the above description; the requested rights are rather determined by the following claims.

**Claims**

1. A non porphyrin compound or a pharmaceutically acceptable salt thereof, suitable for in vitro, in vivo and/or ex vivo use as a diagnosticum and/or pharmaceutical, said compound comprising:

   - a targeting agent for targeting a specific area, such as an organ and/or tissue,
   - a labelling agent L for labelling the targeted area; said targeting agent being connected to the labelling agent L, wherein said targeting agent comprises one or more substituted and/or unsubstituted organic ring compounds.

2. A non porphyrin compound or a pharmaceutically acceptable salt thereof according to claim 1, wherein the targeting agent comprises one or more substituted and/or unsubstituted aryl groups, the non porphyrin compound having the following general formula:

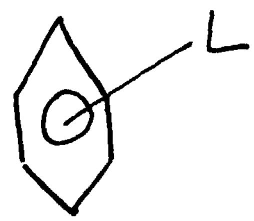

3. Compounds according to claim 2 further comprising a spacing agent S arranged between the targeting agent and the labelling agent L as follows:

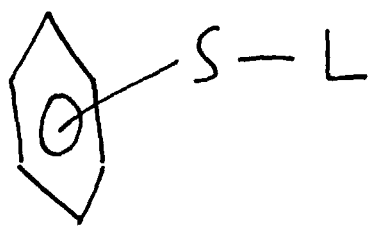

4. Compound according to claims 2 or 3 wherein the targeting agent comprises the following structural formulas A or B:

A

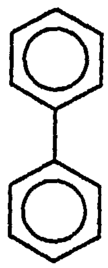

B

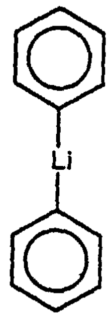

wherein m and p are numerals, wherein m + p $\geq$ 1, wherein in structure B, the aryl rings of the targeting agent are linked by a linking agent Li, wherein the linking agent Li optionally bears a labelling agent L, optionally separated from the linking agent Li by a spacing agent S.

**5.** Compound according to any of the preceding claims wherein the labelling agent comprises one or more of the following:

- one or more radioactive atom(s) (e.g. radioactive iodine, radioactive carbon, radioactive fluorine) covalently bound to the targeting agent;
- dense atom(s), such as stable iodine, covalently bound to the targeting agent;
- one or more radioactive or non-radioactive, homogeneous or heterogeneous metal chelates, preferably with radioactive or non-radioactive ions of an element with atomic number 13, 21-32, 37-39, 42-44, 49 50 or 57-83.

**6.** Compound according to claim 5 wherein the chelator is comprises:

- metal complexing agents based on bisamine-bisthiol, bisamine-bisoxime, monomercapto-triamide, diamide-dithiol, monoamine-monoamide-dithiol, tetramine, monoamine-diamide-monothiol, monoamine-monothioetherdithiol, monoamine-monothiol, monoamide-diamine-monothiol and diphosphine compounds; and
- metal complexing agents having in their structure one or more group(s) with the following structural formulas:

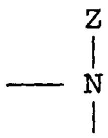

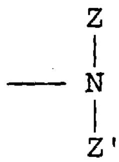

wherein Z stands for a radical selected from the list comprising: phosphonomethyl ($-CH_2PO_3H_2$), carboxy methyl ($-CH_2COOH$) and its derivatives (-C(X)HCOOH), carboxy ethyl ($-CH_2CH_2COOH$) and its derivatives (-C(X)$HCH_2COOH$ and $-CH_2C$(X)HCOOH), wherein X represents an alkyl, aryl or aralkyl group;
wherein Z' stands for hydrogen or a radical selected from the list comprising: the radical Y, phosphonomethyl ($-CH_2PO_3H$), carboxy methyl ($-CH_2COOH$) and its derivatives (-C(X)HCOOH), carboxy ethyl ($-CH_2CH_2COOH$) and its derivatives (-C(X)$HCH_2COOH$ and $-CH_2C$(X)HCOOH), hydroxyethyl ($-CH_2CH_2OH$) and its derivatives (-C(X)$HCH_2OH$ and $-CH_2C$(X)HOH), wherein X represents an alkyl, aryl or aralkyl group;
such as iminoacetic acid (=$N(CH_2COOH)$), iminopropionic acid (=$N(CH_2CH_2COOH_2)$), iminodiacetic acid (-$N(CH_2COOH_2)$), iminodipropionic acid (-$N(CH_2CH_2COOH)_2$), iminoacetic propionic acid (-$N(CH_2CH_2COOH)(CH_2COOH)$), hydroxyethyl iminoacetic acid (-$N(CH_2COOH)(CH_2CH_2OH)$) and hydroxyethyl iminopropionic acid (-$N(CH_2CH_2COOH)(CH_2CH_2OH)$) group(s).

**7.** Compound according to claim 6 wherein the chelator is selected from the group consisting essentially of the following agents having the general formulas F3a-k:

F3a

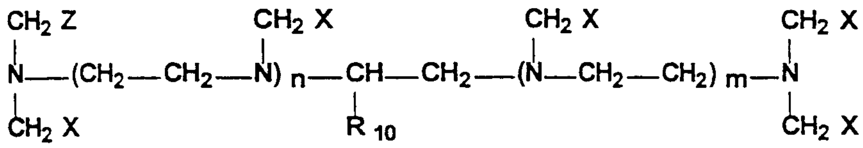

F3b

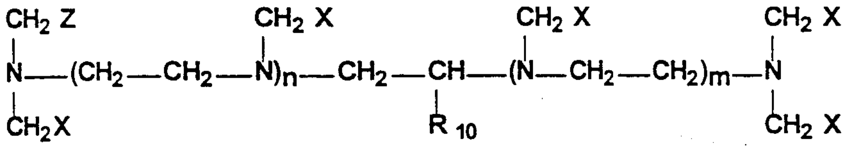

F3c

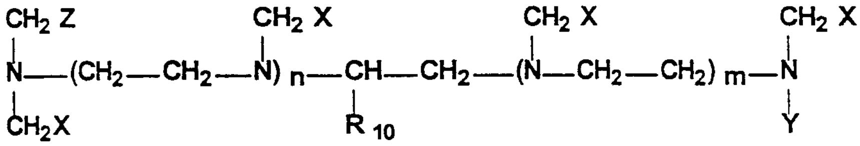

F3d

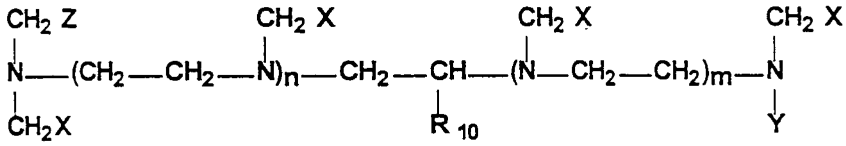

F3e

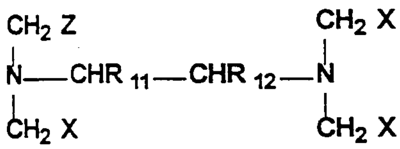

F3f

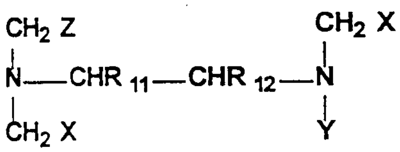

F3g

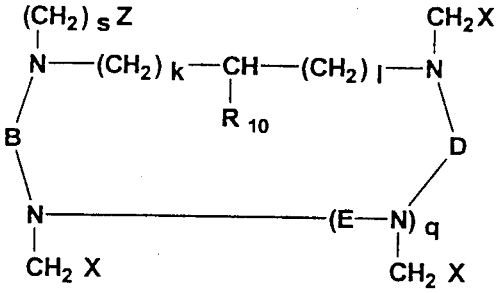

with B, D and E

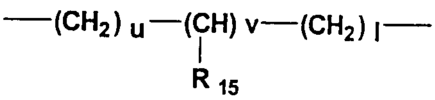

F3h

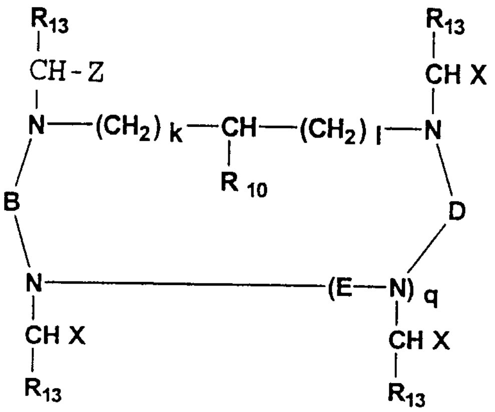

F3i

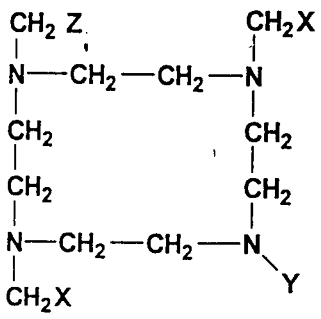

F3j

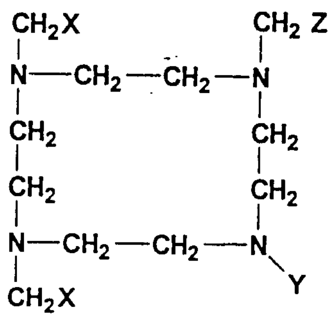

F3k

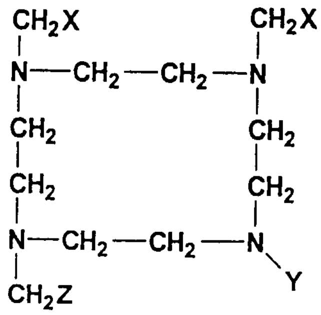

wherein:

- n and m each stand (independently from each other) for the numerals 0, 1, 2, 3 or 4, and the sum of n and m is not greater than 4;
- k stands for the numerals 1, 2, 3, 4 or 5;
- l stands for the numerals 0, 1, 2, 3, 4 or 5;
- q stands for the numerals 0, 1 or 2;
- s stands for the numerals 0 or 1;
- X stands for -COOH or $-PO_3H_2$
- Y stands for a direct link (to the spacing agent or to the targeting agent) or for a straight-chain or branched-chain or cyclic, saturated or unsaturated aliphatic hydrocarbon radical with up to 20 C-atoms optionally substituted

* by one or more hydroxy or lower alkoxy groups,
* by one or more fluorine, chlorine, bromine or iodine atom(s)
* by carboxy, amino, carbamoyl, trifluoromethylgroup(s);
* a saturated or unsaturated 5- or 6-membered ring that optionally contains nitrogen, oxygen, or sulfur atom(s) or a carbonyl group and that is optionally substituted by:
* one or more C1-C6 alkyl, or C1-C6 hydroxyalkyl radical(s)
* optionally hydroxylated or C1-C6 alkoxylated C2-C6 acyl radical(s)
* hydroxy, carbamoyl, carbamoyl-substituted C1-C6 alkyl,
* by one or more fluorine, chlorine, bromine or iodine atom(s)
* a carbamoyl radical substituted at the carbamoyl nitrogen by one or two C1-C6 alkyl radical(s) which can form a ring optionally containing an oxygen atom or a C1-C6 acylamino or C1-C6 alkylamino radical(s)

- B, D and E, which are the same or different, are represented by the formula F3f, wherein:

  * R15 in the meaning of hydrogen or a straight-chain, branched, saturated or unsaturated C1-C20 hydrocarbyl group optionally containing oxygen and/or nitrogen atom(s) and optionally substituted by hydroxy and/or amino group(s);
  * u stands for numerals 0, 1, 2, 3, 4 or 5;
  * v stands for numerals 0 or 1;
  * apart from R15, B, D and E each contain at least 2 and most 5 carbon atoms in their main chain;

- Z stands for the group:

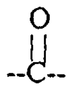

- or the radical X;
- R10 stands for a hydrogen atom or for a direct bond (to linking agent or to targeting agent) or for the substituent Y (see above)
- R11 and R12 together stand for a dimethylene- or trimethylene-methine group optionally substituted by 1-2 hydroxy or 1-3 C1-C4 alkylgroups
- R13 stands for a lower alkyl chain
- Z stands for the group:

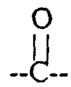

only when R10 and Y are not direct links, wherein, if R10 or Y are direct links Z stands for X and s for the numeral 1 with the proviso that R10 and Y do not mean simultaneously a direct link.

**8.** Compound according to any of the previous claims, wherein the labelling agent comprises:

- a mercaptoacetyl tripeptide such as mercaptoacetyl triglycine (MAG3), a propylene diamine dioxime tetraligand bearing one or more alkyl substituents such as hexamethyl propylene diamine dioxime (HMPAO), ethylene dicysteine, ethylene cysteine cysteamine, cysteinylglycine cysteine, bismercaptoacetyldiaminopropionic acid, bismercaptoacetyldiaminosuccinic acid, N-(mercaptoacetylaminoethyl)-cysteine, dimercaptosuccinic acid, dimercaptopropionic acid, cysteine, cysteamine, diphosphinopropionic acid;
- iminoalkyl acids such as ethylenediaminetetraacetic acid, diethylene triaminopentaacetic acid, triethylene tetramine hexaacetic acid, trans-1,2-cyclohexanediamine tetraacetic acid, 1,4,7,10-tetraazacyclododecane tetraaceticacid, 1,4,7-triazacyclononanetriacetic acid, 1,4,8,11-tetraazatetradecanetetraacetic acid, 1,5,9-triazacyclododecanetriacetic acid,
- analogues thereof in which one or more of the acetic acid groups on the iminogroups are replaced by Y,

wherein Y together with the amino group to which it is attached comprises, among a variety of other compounds: aminoethanol, 2-amino-2-ethyl-1,3-propanediol, 3-methyl-1-butamine, 2-(2-aminoethoxy)ethanol, 1-(3-aminopropyl)imidazole, 4-(3-aminopropyl)morpholine, 1-(3-aminopropyl)-2-pipecoline, 1-(3-aminopropyl)-2-pyrrolidinone, 1-(2-aminoethyl)pyrrolidine, 1-(2-aminoethyl)pyrrolidine, 1-(2-aminoethyl)piperidine, 2-(2-aminoethyl)pyridine, 1-(2-aminoethyl)pyrrolidine, 4-(2-aminoethyl)morpholine, 4-(2-aminoethyl)-1-methylpyrrolidine, (aminomethyl)cyclopropane, 2-(aminomethyl)pyridine, 3-(aminomethyl)pyridine, 2-(aminomethyl)-1-ethylpyrrolidine, furfurylamine, tetrahydrofurfurylamine, 2-(aminomethyl)-tetrahydropyran, 2-(aminomethyl)-1,3-dioxolane, 2-(aminomethyl)-1-methylimidazole,
accordingly suitable examples of such analogues are N-(2-hydroxyethyl)diethylenetriamine N,N',N'',N''' tetraacetic acid, N-(2-hydroxyethyl)ethylenediamine N,N',N'' triacetic acid

- derivatives as defined in claim 7 in which Y and/or R10 comprises radicals such as: benzyl, benzyloxymethyl, 4-carboxymethoxybenzyl, 4-methoxybenzyl, 4-ethoxybenzyl, 4-butoxybenzyl, 4-benzyloxybenzyl, 4-(4-methyloxybenzyloxy)-benzyl; the alkyl substituents methyl, propyl, isopropyl, n-, sec- and tert-butyl, pentyl, cyclopentyl, cyclohexyl, hydroxymethyl, 2-hydroxyethyl, 2-hydroxyethyl-1-(hydroxymethyl)-ethyl, 3-hydroxypropyl, 2-hydroxy-isobutyl, 2,3-dihydroxypropyl, 2-,3- and 4-hydroxybutyl, 2-, 3- and 4-hydroxy-2methylbutyl, 2,3,4-trihydroxybutyl, 2,3,4,5,6-pentahydroxyhexyl.

**9.** Compound according to claim 8, wherein the chelator is bound by a carbon atom or by a carbonyl group to the spacing agent and/or to the targeting agent, wherein optionally part of the carboxylic acid groups can be present as an ester and/or amide, wherein:

- suitable esters being lower alkyl-, aryl- or aralkyl-esters, for example methyl, ethyl, propyl, isopropyl, n-,sec- and tert-butyl, pentyl, phenyl, benzyl, diphenylmethyl, triphenylmethyl esters,
- suitable amides being alkylamides such as methyl, ethyl, propyl,

wherein remaining acidic protons, i.e. those that have not been substituted by the central metal ion, can optionally be replaced completely or partially by cations of inorganic or organic bases, basic aminoacids or aminoacid amides, suitable inorganic counter ions being for example the lithium ion, the potassium ion, the calcium ion, the magnesium ion and especially the sodium ion, suitable cations of organic bases being those of primary, secondary or tertiary amines, such as, for example, ethanolamine, diethanolamine, morpholine, glucamine, N,N-dimethylglucamine, tris(hydroxymethyl)-aminomethane and especially N-methylglucamine, suitable cations of amino acids being, for example, those of lysine, arginine and ornithine as well as the amides of any other acidic or neutral amino acid such as for example lysine methylamide, glycine ethylamide and serine methylamide.

**10.** Compound according to any of the previous claims wherein the spacer S comprises:

- a straight-chain or branched-chain, saturated or unsaturated C1-C20 hydrocarbylene group optionally containing imino, polyethyleneoxy, phenylene, phenyleneoxy, phenylenedioxy, phenyleneimino, amino, hydrazo, hydrazono, ester group(s), oxygen, sulfur and/or nitrogen atom(s) and optionally substituted by halogen atom(s) and/or by functional group(s) such as hydroxy, mercapto, imino, epoxy, oxo, thioxo, sulfo, amino and/or carboxy group(s) and/or by straight-chain or branched-chain, saturated or unsaturated alkyl-, aralkyl-, aryl-radicals which optionally bear halogen atom(s) and/or these functional groups or atoms; and/or
- a grouping or atom such as imino, amino, hydrazo, hydrazono, oxygen, sulfur and/or nitrogen atom; optionally substited by one or more straight-chain or branched-chain, saturated or unsaturated alkyl-, aralkyl-, aryl-radical(s) which optionally bear halogen atom(s) and/or functional groups such as hydroxy, mercapto, imino, epoxy, oxo, thioxo, sulfo, amino and/or carboxy group(s).

**11.** Compound according to any of the previous claims, wherein the linker Li comprises:

- a straight-chain or branched-chain, saturated or unsaturated C1-C20 hydrocarbylene group optionally bearing one or more labeling agents containing imino, polyethyleneoxy, phenylene, phenyleneoxy, phenylenedioxy, phenyleneimino, amino, hydrazo, hydrazono, ester group(s), oxygen, sulfur and/or nitrogen atom(s) and optionally substituted by halogen atom(s) and/or by functional group(s) such as hydroxy, mercapto, imino, epoxy, oxo, thioxo, sulfo, amino and/or carboxy group(s) and/or by straight-chain or branched-chain, saturated or unsaturated alkyl-, aralkyl-, aryl-radicals which optionally bear halogen atom(s) and/or these functional groups or atoms, and/or
- a grouping or atom such as imino, amino, hydrazo, hydrazono, oxygen, sulfur and/or nitrogen atom; optionally substituted by one or more straight-chain or branched-chain, saturated or unsaturated alkyl-, aralkyl-, aryl-radical(s) which optionally bear halogen atom(s) and/or functional groups such as hydroxy, mercapto, imino, epoxy, oxo, thioxo sulfo, amino and/or carboxy group(s).

**12.** Compound according to claim 10 or 11, wherein the linking and/or the spacing agent(s) comprise one of the following groups as such and/or a hydrocarbylene chain, comprising one or more of the following groups:

-NH- and N' alkyl, aryl and/or aralkyl derivatives,
-O-,
-S-,
-CO-O- and -O-CO-,
-(O-$CH_2CH_2$)poly-,
-NH-CO- and -CO-NH- and N' alkyl, aryl and/or aralkyl derivatives,
-NH-NH- and N- an/or optionally N'-alkyl, aryl and/or aralkyl derivatives,
-C6H4-,
-C6H4-NH-,
-C6H4-O-,
-O-C6H4-O-,
-($C4NH_3$)- 2,5 pyrrole biradical and optionally 3- and/or 4- and/or N-substituted derivatives;
-($C4SH_3$)- 2,5 thiophene biradical and optionally 3- and/or 4-substituted derivatives;

**13.** Compound according to any of the previous claims, wherein the linking agent comprises a carbon atom with an -ylidene substituent having the general formula:

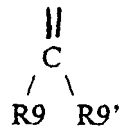

where R9 and R9' are equal or different and stand for

- hydrogen
- a straight chain or branched chain, saturated or unsaturated alkyl-, aryl-, aralkyl- radical optionally containing imino, polyethyleneoxy, phenylene, phenyleneoxy, phenylenedioxy, phenyleneimino, amino, hydrazo, hydrazono, ester group(s), oxygen, sulfur and/or nitrogen atom(s) and optionally substituted by halogen atom(s) and/or by functional group(s) such as hydroxy, mercapto, imino, epoxy, oxo, thioxo, sulfo, amino and/or carboxy group(s) and/or by straight-chain or branched-chain, saturated or unsaturated alkyl-, aralkyl-, aryl-radicals which optionally bear halogen atom(s) and/or these functional groups, wherein:
- R9 and R9' together with the -ylidene C-atom optionally form a 5 or a 6-membered ring that is optionally substituted as above, wherein examples of suitable cyclic -ylidene substituents are:

3-carboxy-4-oxo-2,5-cyclohexadien-1-ylidene,
3-carboxy-4-oxo-5-methyl-2,5-cyclohexadien-1-ylidene,
3-sulfo-4-imino-2,5-cyclohexadien-1-ylidene.

**14.** Compound according to any of the claims 10-13, wherein the linking and/or the spacing agent(s) is/are further derivatised by attachment of one or more bio-molecules, the attachment being achieved by, substitution of suitable group(s) or atom(s) of the linking and/or spacing agent(s) such as for example sec-amino, aryl, halogen(s) and/or via the optionally available substituents mentioned in claims 8, 9, 10, 11 (e.g. hydroxy, mercapto, epoxy, amino, carboxy group).

**15.** Compound according claim 14, wherein the biomolecules include: bile salts and their derivatives cholesterol, cholestane, hormones, biotine and peptides such as glutathione, sugars and oligomeric sugar derivatives.

**16.** Compound according to any of the claims 10-15, wherein the linking agent or the spacing agent is selected from the group consisting essentially of:

$-(CH_2)n-$ (n=1-10),
$-CH_2-O-(CH_2)_2-O-CH_2-$,
$-(CH_2-O-CH_2n-$ (n=1-3),
$-CH_2-S-CH_2-$,
-S-, -SO-
$-SO_2-$,
-CO-,
-CHOH-,
$-C(-C_6H_5)(OH)-$,
$-CH(-C_6H_5)-$,
-NH-NH-,
$-NH-N(CH_2CHOH)-$,
$-NH-N(CH_2COOH)-$,
$-NH-N(-(CH_2)n-CH_3)-$ (n=0,1),
$-NH-N(-C_6H_5)-$,
$-N(-C_6H_5)-N(-C_6H_5)-$,
$-NH-N(-CH_2-C_6H_5)-$
$-N(-CH_2-C_6H_5)-N(-CH_2-C_6H_5)-$,
-CO-NH-NH-,
$-CO-NH-N(CH_2CHOH)-$,
$-CO-NH-N(CH_2COOH)-$,
$-CO-NH-N(-(CH_2)n-CH_3)-$ (n=0,1),
$-CO-NH-N(-C_6H_5)-$,
$-CO-N(-C_6H_5)-N(-C_6H_5)-$,
$-CO-NH-N(-CH_2-C_6H_5)-$,
$-CO-N(-CH_2-C_6H_5)-N(-CH_2-C_6H_5)-$,
$-N(-(CH_2)n-CH_3)-N(-(CH_2)n-CH_3)-n=0,1)$,
$-CHOHCH_2-O-CH_2CHOH-$,
$-CHOHCH_2-O-(CH_2)n-O-CH_2CHOH-$ (n=4),
$-(CH_2)_2-SO_2-(CH_2)_2-$,
$-(CH_2CH_2-O)n-(CH_2)_2-$ (n=4),
$-NHCO-(CH_2)n-CONH-$ (n=1-4),
$-N(CH_3)CO-(CH_2)n-N(CH_3)CO-$ (n=1-2),
$-N(CH_3)CO-CH_2-O-CH_2-N(CH_3)CO-$,
$-NHCO-(CH_2)n-NHCO-$ (n=1, 2, 4),
$-CONH-(CH_2)n-NHCO-$ (n=2) ,
-CONH-NHCO-,
$-NH-NH-CO-(CH_2)n-CO-NH-NH-$
$-CON(CH_3)-N(CH_3)CO-$,
$-CONH-CH(CO_2H)-(CH_2)n-NHCO-$ (n=5),
$-CONH-(C_nH_{2n}-O)_m-CnH_{2m}-NHCO-$ (n=2 or 3, m=1-5),
$-CON(CH_3)--(C_nH_{2n}-O)_m-C_nH_{2m}-N(CH_3)-CO-$ (n=2 or 3, m=1-5),
$-CONH-CH2-(CH_2CH_2-O)_n-(CH_2)_3-NHCO-$ (n=4),
$-CON(CH_3)-(CH_2CH_2-O)n-(CH_2)_2-N(CH_3)CO-$ (n=2, 3, 5),
$-CON(CH_3)-CH2-(CH_2CH_2-O)n-(CH_2)_3-N(CH_3)CO-$ (n=4),
$-(CH_2)_2-NH-$,
$-(CH_2)_2-O-C_6H_4-CH_2-$,
$-(CH_2)_3-O-C_6H_4-CH_2-$,
$-CH_2-CHOH-CH_2-O-C_6H_4-CH_2-$,
$-C(=NH)-O-C_6H_4-CH_2-$,
$-(CH_2)_4-NH-CO-CH_2-C_6H_4-CH_2-$,
$-(CH_2)_4-NH-CO-(CH_2)_8-$,
$-(CH_2)_4-NH-CH_2-CHOH-CH_2-C_6H_4-CH_2-$,
$-CH_2-CO-NH-(CH_2)_3-O-CH_2-$,
$-CH_2-CO-NH-NH-$,
$-CH_2-CO-NH-(CH_2)_2-$,
$-CH_2-CO-NH-(CH_2)_{10}-$,
$-CH_2-CO-NH-(CH_2)_2-S-$,

$-CH_2-CO-NH-(CH_2)_3-NH-$,
$-(CH_2)_3-NH-$,
$-CH_2-NH-C(=S)-NH-C_6H_4-CH_2-$,
$-(CH_2)_2-NH-CO-CH_2-(CH_2CH_2-O)n-O-CH_2-$ (N=3,4),
$-CH_2-(N(CH_2)nN)CH_2-$, (piperazine n=4, homopiperazine n=5),
$-(N(-CH_2-)nN)CH_2-$, (piperazine n=4, homopiperazine n=5),
$-(N(-CH_2-)nN)-$, (piperazine n=4, homopiperazine n=5),
$-CH_2-NH-C(=O)-NH-CH_2-$,
2,5 thiophene bi-radical and 3 and/or 4 substituted derivatives;
2,5 pyrrole bi-radical and 3 and/or 4 substituted derivatives.

**17.** Compound according to any of the previous claims wherein the organic compound(s) of the targeting agent comprise one or more of the following groups:

acridine,
anthracene,
azulene,
benzene,
benzofuran,
benzoxazole,
imidazole,
indol,
indolenine,
indolizine,
indoxazine,
isobenzofuran,
isoindazole,
isoindene,
isoquinoline,
isothionaphthene,
naphthalene,
naphtyridine,
phenanthrene,
phenanthridine,
quinoline,
retronecene,
thionaphthene,
thaliporphyne,
and (de)hydro-derivatives such as for example
dehydronaphtalene,
dehydroanthracene,
dehydroisoquinoline,
dihydroimidazole,
tetrahydroquinoline,
hydroindolizine.

**18.** Compound according to any of the previous claims, wherein the one or more aryl groups of the targeting agent is/are substituted with one or more of the following groups and/or atoms:

- XH wherein X represents an oxygen, sulfur or nitrogen atom and H a hydrogen atom;
- XR4 wherein X represents an oxygen or sulfur and R4 a group as explained below;
- R1
- R2, being selected from the group consisting essentially of:

    * the halogens;
    * the nitro group ($-NO_2$);
    * the cyano group (-R-CN);
    * the sulfamino group ($-NH-SO_3H$);

* the allophanoyl radical (-CO-NH-CO-$NH_2$);
* the guanidino radical (-NH-C(=NH)-$NH_2$ );
* the guanyl radical (-C(=NH)-$NH_2$ )
* the radical -R-N(R5)(R5'), being selected from the group consisting essentially of: primary, secondary and tertiary amines, amides, carbamates, ureides and sulfonamides;
* the radical -R-CO-A, wherein A represents:
    - hydrogen,
    - the -OH radical,
    - a R3 radical, the -R-CO-R3 radical being selected from the group consisting of carbonyls,
    - a -OR4 radical,
    - a -N(R6)(R6') radical, the -R-CO-N(R6)(R6') radical being selected from the group consisting essentially of amides, N-substituted amides, hydrazynocarbonyls; N and/or N'-substituted hydrazynocarbonyls;
* the radical -R-$SO_2$-B, wherein B represents,
    - the -OH radical,
    - a -N(R7)(R7') radical, the -R-$SO_2$-N(R6)(R6') radical being selected from the group consisting essentially of N-substituted or not substituted sulfonamides

wherein R is absent (direct bond) or wherein R represents a straight-chain, branched-chain, saturated or unsaturated C1-C20 hydrocarbylene group optionally containing imino, polyethyleneoxy, phenylene, phenyleneoxy, phenylenedioxy, phenyleneimino, amino, hydrazo, ester group(s), oxygen, sulfur and/or nitrogen atom(s) and optionally substituted by functional groups such as hydroxy, mercapto, imino, epoxy, oxo, thioxo, amino and/or carboxy group(s) and/or by straight-chain or branched-chain, saturated or unsaturated alkyl-, aralkyl-, aryl-radicals which optionally bear halogen atom(s) and/or functional groups such as those mentioned above;
wherein R1 and R3 represent for a straight-chain, branched-chain, saturated or unsaturated C1-C20 hydrocarbyl radical optionally containing imino, polyethyleneoxy, phenylene, phenyleneoxy, phenylenedioxy, phenyleneimino, amino, hydrazo, ester group(s), oxygen, sulfur and/or nitrogen atom(s) and optionally substituted by functional groups such as hydroxy, mercapto, imino, epoxy, oxo, thioxo, amino and/or carboxy group(s) and/or by straight-chain or branched-chain, saturated or unsaturated alkyl-, aralkyl-, aryl-radicals which optionally bear halogen atom(s) and/or functional groups such as those mentioned above;
wherein R4 represents a C1-C6 hydrocarbon radical, a benzyl radical or a glucoside moiety, optionally substituted by functional groups such as hydroxy, mercapto, imino, epoxy, oxo, thioxo, amino and/or carboxy group(s) and/or by straight-chain or branched-chain, saturated or unsaturated alkyl-, aralkyl-, aryl-radicals which optionally bear halogen atom(s) and/or functional groups such as those mentioned above;
wherein R5 and R5' ( -R-N(R5)(R5') ) represent hydrogen; and/or a straight-chain or branched-chain or cyclic, saturated or unsaturated aliphatic alkyl-, aryl-, aralkyl-, acyl-, carboxyalkyl-, carboxyaryl-, carboxyaralkyl-, aminocarbonyl-, sulfonylalkyl-, sulfonylaryl-, sulfonylaralkyl-radical, optionally substituted by one or more functional group(s) and/or atom(s) such as: hydroxy, mercapto, halogenated or not halogenated alkyl and alkoxy groups, fluorine, chlorine, bromine or iodine atom(s), carboxy, primary, secondary and tertiary amino and sulfon group(s), carboxylic ester group(s), carbonyl group(s), formyl group(s) and optionally containing additional N, O, S, atom(s), accordingly -R-N(R5)(R5') radicals comprise:

- primary amines (-R-$NH_2$) wherein both R5' and R5 represent hydrogen;
- secondary amines (-R-NHR5) wherein R5' is hydrogen and R5 represents an alkyl-, aryl-, aralkyl-radical;
- tertiary amines (-R-N(R5)(R5')) wherein both R5' and R5 represent an alkyl-, aryl-, aralkyl-radical;
- cyclic amines (-R-N(R5)(R5')) wherein R5' and R5 together with the amino nitrogen, may represent a saturated or unsaturated 5- or 6-membered ring that optionally contains additional nitrogen, oxygen, sulfur atom(s) and carbonyl groups and that is optionally substituted by:
    * one or more optionally halogenated C1-C6 alkyl, or C1-C6 hydroxyalkyl radical(s)
    * an optionally hydroxylated or C1-C6 alkoxylated C2-C6 acyl radical
    * hydroxy, carbamoyl, carbamoyl-substituted C1-C6 alkyl,
    * a carbamoyl radical substituted at the carbamoyl nitrogen by one or two C1-C6 alkyl radical(s) which can form a ring optionally containing an oxygen atom or C1-C6 acylamino or C1-C6 alkylamino radical(s)

- amides (-R-NH-CO-) and N-alkylated amides (-R-NR5'-CO-) wherein R5 stands for an acylalkyl-, acylaryl-, acylaralkyl-radical and R5' represents hydrogen and an alkyl-radical respectively;
- carbamates (-R-NH-CO-O-) and N-alkylated carbamates (-R-NR5'-CO-O-) wherein R5 represents a carboxyalkyl-, carboxyaryl-, carboxyaralkyl-radical and R5' represents hydrogen and an alkyl-radical, respectively;
- ureides (-R-NH-CO-N(-)(-)) and N-alkylated ureides (R-NR5'-CO-N(-)(-)) wherein R5 represents an aminocarbonyl-radical and R5' stands for hydrogen and an alkyl-radical, respectively;
- sulfonamides (-R-NH-$SO_2$-) wherein R5' represents hydrogen and R5 stands for a sulfonylalkyl-, sulfonylaryl-, sulfonylaralkyl-radical;

wherein R6 and R6' ( -R-CO-N(R6)(R6') ) represent hydrogen; and/or a straight-chain or branched-chain or cyclic, saturated or unsaturated aliphatic alkyl-, aryl-, aralkyl-, aminoalkyl-, aminoaryl-, aminoaralkyl- radical, optionally substituted by one or more functional group(s) and/or atom(s) such as: hydroxy, mercapto, halogenated or not halogenated alkyl and alkoxy groups, fluorine, chlorine, bromine or iodine atom(s), carboxy, primary, secondary and tertiary amino and sulfon group(s), carboxylic ester group(s), carbonyl group(s), formyl group(s) and optionally containing additional N, O, S, atom(s), accordingly radicals -R-CO-N(R6)(R6') comprise:

- amides (-R-CO-NH-R6) and N-substituted amides (-R-CO-NR6'-R6) wherein R6 stands for alkyl-, aryl-, aralkyl-radical and R6' stands for hydrogen and an alkyl-radical respectively;
- cyclic amides (-R-CO-N(R6)(R6')) wherein R6' and R6 together with the aminocarbonyl nitrogen, may represent a saturated or unsaturated 5- or 6-membered ring that optionally contains additional nitrogen, oxygen, sulfur atom(s) and carbonyl group(s) and that is optionally substituted by:

  * one or more optionally halogenated C1-C6 alkyl, or C1-C6 hydroxyalkyl radical(s)
  * an optionally hydroxylated or C1-C6 alkoxylated C2-C6 acyl radical
  * hydroxy, carbamoyl, carbamoyl-substituted C1-C6 alkyl,
  * a carbamoyl radical substituted at the carbamoyl nitrogen by one or two C1 C6 alkyl radical(s) which can form a ring optionally containing an oxygen atom or a C1-C6 acylamino or C1-C6 alkylamino radical(s);

- hydrazinocarbonyls (-R-CO-NH-NH-, according to -R-CO-N(R6)(R6')) wherein R6' stands for hydrogen and R6 stands for an aminoalkyl-, aminoaryl-, aminoaralkyl- radical and; N'-substituted hydrazynocarbonyls wherein R6 stands for aminobisalkyl-, amino(alkyl)(aralkyl)-, aminobisaralkyl-, amino(aralkyl)(aryl)-, aminobisaryl- or an amino(alkyl)(aryl)-radical, the above hydrazynocarbonyls wherein R6' stands for an alkyl-, aralkyl-or aryl-radical;

wherein R7 and R7' ( -R-$SO_2$-N(R6)(R6') ) represent hydrogen; and/or a straight-chain or branched-chain or cyclic, saturated or unsaturated aliphatic alkyl-, aryl-, aralkyl-radical, optionally substituted by one or more functional groups such as for example hydroxy, alkoxy, fluorine, chlorine, bromine or iodine atom(s), carboxy, primary, secondary and tertiary amino group(s), carboxylic ester group(s), carbonyl group(s), formyl group(s) and optionally containing additional N, O, S, atom(s).

**19.** Compound according to any of the previous claims, wherein the substituents on the targeting agent comprise the following series for R and R2 substituents either grouped under the heading of the formula R-N(R5)(R5') or grouped under the heading of the formula R-COA, according to claim 16, wherein suitable R-N(R5)(R5') groups comprise the following, wherein:

- if both R5 and R5' stand for H, this refers to primary amines, either as an amino function directly attached to the targeting agent or separated from it by the bifunctional radical -R-,
- optionally R5' and R5 representing hydrogen and an alkyl group (secondary amines) or two alkyl groups (tertiary amines), suitable alkyl groups being there the groups methyl, ethyl, 2-hydroxyethyl, 2-hydroxy-1-(hydroxymethyl)-ethyl, 1-(hydroxymethyl)-ethyl, propyl, isopropyl, 2-hydroxypropyl, 2,3-dihydroxypropyl, butyl, isobutyl, isobutenyl, 2-hydroxybutyl, 3-hydroxybutyl, 4-hydroxybutyl, 2-, 3- and 4-hydroxy-2-methylbutyl, 2- and 3-hydroxyisobutyl, 2-, 3-,4-trihydroxybutyl, 1,2,4-trihydroxybutyl, pentyl, cyclopentyl, cyclohexyl, 2,3,4,5,6-pentahydroxyhexyl and 2-methoxyethyl, amines where R5 together with the N-atom and R5' being represented by N-R 1-glucamine and its N-alkyl derivatives, wherein R5' stands for example for methyl or 2-hydroxyethyl,
- heterocyclic amine radicals, wherein a 5- or 6-membered ring is formed by R5 and R5' together with the amino nitrogen, that is optionally saturated, unsaturated, and/or substituted and optionally contains additional nitrogen, oxygen, sulfur atom(s) and carbonyl group(s), wherein the heterocyclic moiety is optionally substituted by

the following groups, hydroxy group(s); C1-C6 alkyl group(s), for example methyl, ethyl, propyl isopropyl, butyl; C1-C5 hydroxyalkyl group(s), for example hydroxymethyl, hydroxyethyl; C1-C6 alkoxy group, for example methoxy and ethoxy, wherein further optional substituents on the heterocyclic ring are a carbamoyl group bound to the heterocyclic compound either directly or separated from it by a C1-C6 alkylene group, for example methylene, ethylene, propylene and is optionally substituted on the carbamoyl nitrogen by one or two C1-C6 alkyl radical(s), for example methyl, ethyl, propyl, isopropyl which optionally form a ring for example, a pyrrolidine or piperidine ring, wherein the amino nitrogen can optionally be an element of a morpholine ring, a C1-C6 alkylated or a C1-C6 acylated (e.g. by alkanoyl) primary or secondary amino group, for example the methyl, ethyl acetyl, propionyl amino groups, wherein when the heterocyclic compound is substituted the total number of substituents is 1 to 3, wherein suitable heterocyclic amine substituents comprise the following groups: pyrrolyidinyl, piperidyl, pyrazolidinyl, pyrrolinyl, pyrazolinyl, piperazinyl, morpholinyl, imadazolidinyl, oxazolidinyl and thiazolidinyl ring, wherein suitable amides are N-unsubstituted amides (-R-NH-CO-) and N-substituted amides (-R-NR5'-CO-) wherein R5 stands for an acylalkyl-, acylaryl-, acylaralkyl-radical and R5' stands for hydrogen and an alkyl-radical respectively and comprise the following: acetamido, hydroxyacetamido, trifluoroacetamido, N-methyl-, N-ethyl-, N-hydroxyethyl-, N-dihydroxypropylacetamido, acetoacetamido; wherein if R-chain stands for methylene preferred amides are methyl-acetamido (-CH2-NHCO-$CH_3$), methyl-hydroxyacetamido (-CH2-NHCO-$CH_2OH$);

- amides derived from saturated and unsaturated (fatty) acids wherein R5 stands for adipyl (-CO-$(CH_2)_4CO_2$), butyryl (-CO-$(CH_2)_2CH_3$), caproyl (-CO-$(CH_2)_4CH_3$), capryl (-CO-$(CH_2)_8CH_3$), caprylyl (-CO-$(CH_2)_6CH_3$), hydantoyl (-CO-$CH_2NHCONH_2$), isovaleryl (-CO-$CH_2CH(CH_3)_2$), lactyl (-CO-$CHOHCH_3$), lauroyl (-CO-$(CH_2)_{10}CH_3$), levulinyl (-CO-$(CH_2)_2COCH_3$), mandelyl (-CO-CH(OH)$C_6H_5$), methacrylyl (-CO-$(CH_3)C=CH_2$, myristyl (-CO-$(CH_2)_{12}CH_3$), oleyl (-CO-$(CH_2)_7CH=CH(CH_2)_7CH_3$, palmityl (-CO-$(CH_2)_{14}CH_3$), pelargonyl (-CO-$(CH_2)_7CH_3$), propionyl (-CO-$CH_2CH_3$), sorbyl (-CO-CH=CHCH=$CHCH_3$), stearyl (-CO-$(CH_2)_{16}CH_3$), succinamyl (-CO-$CH_2CH_2CONH_2$), toluyl (-CO-$C_6H_5CH_3$), valeryl (-CO-$(CH_2)_3CH_3$),
- amides wherein R5' stands for H, and where the acyl-group is derived from either an animo acid or an amino acid derivative or a peptide, preferably being the acylradicals: β-glutamyl or γ-glutamyl, β-alanyl, glycyl, N-benzoylglycyl, (hippuryl), isoleucyl, leucyl, methionyl, seryl, tyrosyl, valyl, the acyl derative of the tripeptide glutathione and its disulfide derivatives with a blocked thiol group, wherein the
- N-substituted or non substituted amides, comprising: 2-ketogulonamido (-NR5'CO-CO$(CHOH)_3CH_3$), D-gluconamido (-NR5'CO-$(CHOH)_4CH_2OH$) and in particular gluconanilides (-NR5'CO-$(CHOH)_4COH$), wherein the amido functions such as those in which the acylchain R5 together with the amide nitrogen substituent R5' form a ring such for example a buterolactamido (or methylbuterolactamido where R stands for methylene) or a succinimido substituent,
- carbamates represented by (-NH-CO-O-) and N-substituted carbamates represented by (-NR5'-CO-O-) wherein R5 stands for a carboxyalkyl-, carboxyaryl-, carboxyaralkyl-radical and R5' stands for hydrogen and an alkyl-radical, respectively, preferably comprising the following: -NHCO-$OCH_3$, -NHCO-$OCH_2CH_3$, -NHCO-$OCH_2C_6H_5$); the carbamates of the type -NHCO-O-Z where Z preferably represents 1-L-sorbose, 6-D-galactose, 6-D-glucose,
- ureides represented by (-NH-CO-N(-)(-)) and N-substituted ureides (-NR5'-CO-N(-)(-)), wherein R5 stands for an aminocarbonyl-radical and R5' stands for hydrogen and an alkyl-radical, respectively comprising the following:
- ureides in which the carbamoyl radical is represented by the formula RCONR6R6' with R standing for a direct link,
- ureides in which, apart from N-substitution, the carbamoyl nitrogen, viz. N', is also substituted;
- ureides of the type -NR5'CO-NZ'Z'' where R5' stands for hydrogen, Z'' represents hydrogen or methyl and NZ' represents 1-amino-1 deoxy-D-glucitol, 2-amino-2-deoxy-D-glucose or 2-amino-2-deoxy-D-glucitol, wherein
- suitable R-COA groups preferably comprise the following:
- a formyl group wherein (A stands for H) carboxy compounds wherein (A stands for OH) wherein R is absent or R represents methylene, ethylene and propylene to yield the corresponding carboxy, carboxymethyl, carboxyethyl, carboxypropyl groups, respectively, wherein the acidic H-atom of these compounds is optionally replaced by an organic or inorganic counter ion,
- carboxylic esters wherein (A stands for R4) wherein R4 represents the hydrocarbons with 1-6, preferably 1-4 C-atoms, preferably being methyl, ethyl, propyl, isopropyl, n-, sec. or tert.-butyl, isobutyl, pentyl and hexylradicals,
- esters wherein R4 represents glycoside moieties and their derivatives for example β-D-glucosyl, 1-glucopyranosyl, glucuronosyl and its amide, β-D-maltosyl,

wherein A optionally represents radical -N(R6)(R6') being amides (-CO-NH-R6) and N-substituted amides (-CO-NR6'-R6) wherein R6 represents an alkyl-, aryl-, aralkyl-radical and R6' represents hydrogen and a substituent, viz. an alkyl-, aryl- or aralkyl-radical respectively comprising the following:

- amides and/or N-substituted amides wherein R6' and/or R6 represents saturated, unsaturated, straight chain or branched chain or cyclic hydrocarbons optionally being substituted by 1 or more hydroxy, O-acetylhydroxy or lower alkoxy groups, wherein the lower alkoxy groups preferably comprise 1-4 C-atoms and in particular methoxy and ethoxy groups, optionally substituted alkylgroups are preferably selected from the following groups, methyl, ethyl, 2-hydroxyethyl, 2-hydroxy-1-(hydroxymethyl)-ethyl, 1-(hydroxymethyl)-ethyl, propyl, isopropyl, 2-hydroxypropyl, 2,3-dihydroxypropyl, butyl, isobutyl, isobutenyl, 2-hydroxybutyl, 3-hydroxybutyl, 4-hydroxybutyl, 2-, 3- and 4-hydroxy-2-methylbutyl, 2- and 3-hydroxyisobutyl, 2,3,4,-trihydroxybutyl, 1,2,4-trihydroxybutyl, pentyl, cyclopentyl, cyclohexyl, 2,3,4,5,6-pentahydroxyhexeyl and 2-methoxyethyl,
- carbamyl compounds wherein R6 together with the N-atom and R6' being H represent 1-glucamine with is N-alkyl derivatives, as for example R6=methyl, 2-hydroxyethyl; the carbamyl compounds with R6'=H and R6 represents for example a 2-glucose derivative (R6=$-CHZ-(CHOH)_3-CH_2OH$, with Z preferably being a formyl group or $-CH_2OH$),
- amides wherein R6 and R6' together with the N-atom represent an amino acid or a small peptide, for example lysine bound either by its $\alpha$- or $\varepsilon$-aminogroup, the tripeptide glutathione and its disulfide derivatives or derivatives with e.g. a blocked thiol group, wherein if R6' stands for a hydrogen atom, R6 represents a C6-C10 aryl or C6-C10- aryl-C1-C6 alkyl group, for example being a phenyl or benzyl group, optionally substituted by one or more (up to three) di-C1-C6 alkylamino groups, by one or more (up to three) C1-C6 alkoxy groups,
- heterocyclic amide radicals, wherein a 5- or 6- membered ring is formed by R6 and R6' together with the carbamoyl nitrogen, which can be saturated, unsaturated, and/or substituted and optionally contain additional nitrogen, oxygen, sulfur atom(s) and carbonyl group(s), wherein the heterocyclic moiety (is optionally) substituted by hydroxy group(s); by C1-C6 alkyl group(s), for example methyl, ethyl propyl isopropyl, butyl; by C1-C5 hydroxyalkyl group(s), for example hydroxymethyl, hydroethyl; by C1-C6 alkoxy group, for example methoxy ethoxy, wherein optionally the heterocyclic ring further comprises the following substituents: a carbamoyl group that is bound to the heterocyclic compound either directly or separated from it by a C1-C6 alkylene group, for example methylene, ethylene, propylene and is optionally substituted on the carbamoyl nitrogen by one or two C1-6 alkyl radical(s), for example methyl, ethyl, propyl, isopropyl which optionally form a ring such as a pyrrolidine or piperidine ring, wherein the carbamoyl nitrogen is optionally an element of a morpholine ring, an optionally C1-C6 alkylated or a C1-C6 acylated (e.g. by alkanoyl) primary or secondary amino group, such as for example the methyl, ethyl, acetyl, propionyl amino groups, wherein, if the heterocyclic compound is substituted the total number of substituents is preferably 1 to 3, wherein suitable heterocyclic compound include, the pyrrolyidinyl, piperidyl, pyrazolidinyl, pyrrolinyl, piperazinyl, morpholinyl, imadazolydinyl, oxazolidinyl and thiazolidinyl ring,
- R-COA where A represents the radical -N(R6)(R6') preferably being hydrazinocarbonyls (-CO-NH-NH-) wherein R6 represents an aminoalkyl-, aminoaryl-, aminoaralkyl- radical and R6' represents hydrogen, comprising the following:
- hydrazinocarbonyls in which the alkyl, the aryl and the aralkyl moiety of the corresponding aminoalkyl-, aminoaryl-, aminoaralkyl- radical preferably represents an acyl radical such as adipyl ($-CO-(CH_2)_4CO_2$), anisyl ($-CO-C_6H_4-O-CH_3$), aminobenzoyl ($-CO-C_6H_4-NH_2$), benzoyl ($-CO-C_6H_5$), hydroxybenzoyl ($-CO-C_6H_4-OH$), hydrocynnamoyl ($-CO-(CH_2)_2-C_6H_5$), enanthyl ($-CO-(CH_2)_5CH_3$), isobutyryl ($-CO-CH(CH_3)_2$), butyryl ($-CO-(CH_2)_2CH_3$), caproyl ($-CO-(CH_2)_4CH_3$), capryl ($-CO-(CH_2)_8CH_3$), caprylyl ($-CO-(CH_2)_6CH_3$), hydantoyl ($-CO-CH_2NHCONH_2$), isovaleryl ($-CO-CH_2CH(CH_3)_2$), lactyl (-CO-CHOHCH), lauroyl ($-CO-(CH_2)_{10}CH_3$), levulinyl ($-CO-(CH_2)_2COCH_3$), mandelyl ($-CO-CH(OH)C_6H_5$), methacrylyl ($-CO-(CH_3)C=CH_2$), myristyl ($-CO-(CH_2)_{12}CH_3$), oleyl ($-CO-(CH_2)_7CH=CH(CH_2)_7CH_3$), palmityl ($-CO-(CH_2)_{14}CH_3$), pelargonyl ($-CO-(CH_2)_7CH_3$), propionyl ($-CO-CH_2CH_3$), sorbyl (-CO-CH=CHCH=CHCH$_3$), stearyl($-CO-(CH_2)_{16}CH_3$), succinamyl ($-CO-CH_2CH_2CONH_2$), toluyl ($-CO-C_6H_5CH_3$), valeryl ($-CO-(CH_2)_3CH_3$),
- hydrazinocarbonyls in which the aminoalkyl-, the aminoaryl-, or the aminoaralkyl-radical stands, for example, for 2-aminopyridine, 4-aminobenzoic acid, 2-aminafluorobenzoic acid, 4-aminoacetanilide, 1-aminophthalazine, 2-amino-2-imidazole, 2-aminopyridine, 2-amino-4-(trifluoromethyl)pyridine, 3-hydroxybenzylamine, 7-chloro-4-aminoquinoline, amino-N-benzyl, -N-diphenyl, -N-trityl, aminoalkyl- and hydroxylated aminoalkyl-radicals such as amino-2-hydroxy ethyl.

**20.** Compound according to any of the preceding claims as optionally stabilized (for example by antioxidants) and optionally neutralized (by organic or inorganic bases such as sodium hydroxide and methylglucamine) having the following formulas and labelled by chelation of one or more radioactive or non-radioactive ions of an element with atomic number 13, 21-32, 37-39, 42-44, 49, 50 or 57-83; as for example Mn, Fe, Gd, $^{99m}$TC, $^{111}$In, $^{67}$Ga, $^{90}$Y, $^{188}$Re, $^{186}$Re and $^{163}$Dy, wherein in the formulas R stands for one of the following chelating groups, viz.

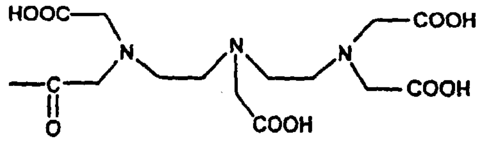

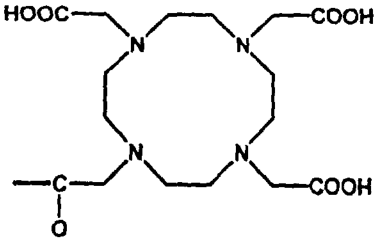

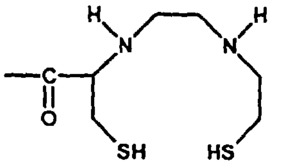

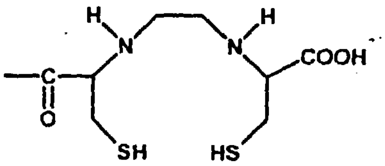

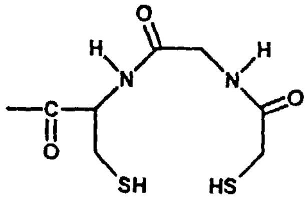

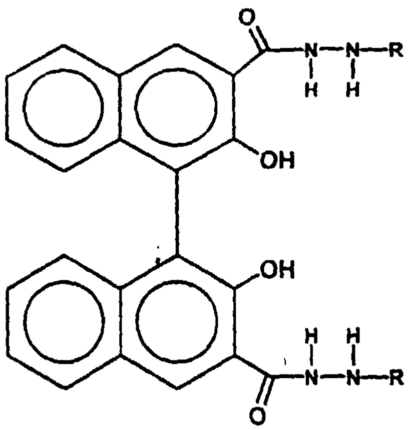

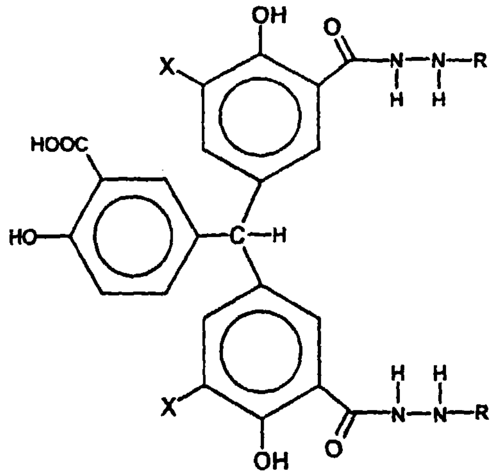

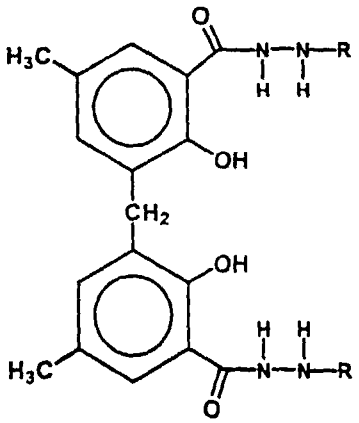

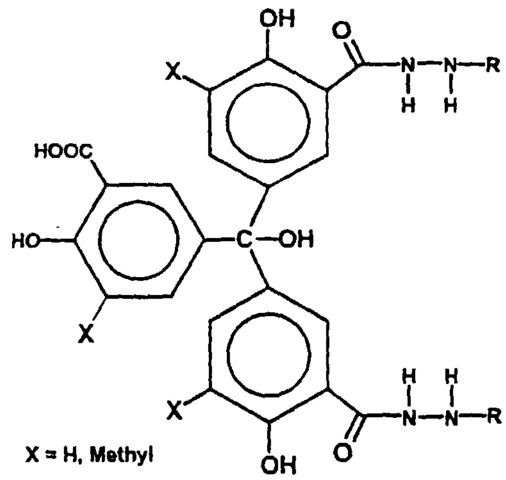

X = H, Methyl

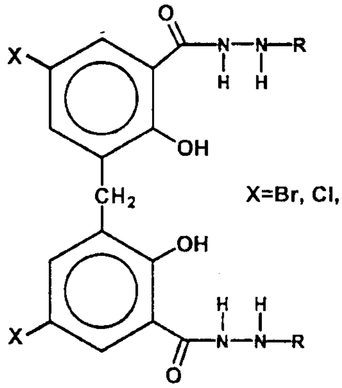

X=Br, Cl,

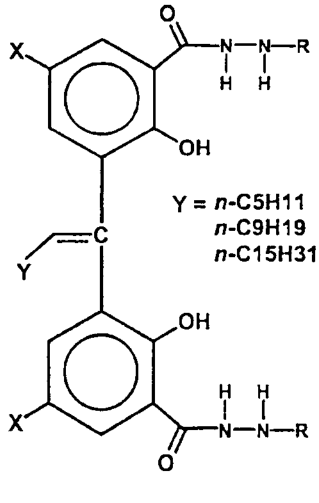

Y = *n*-C5H11 X = Br, Cl,
*n*-C9H19
*n*-C15H31

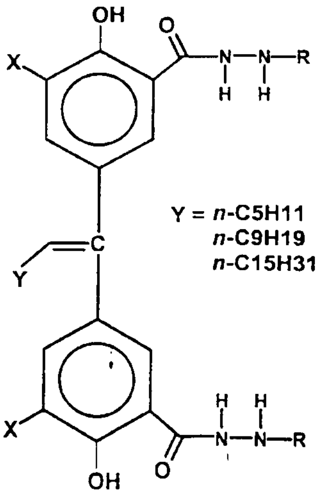

Y = *n*-C5H11 X = Br, Cl,
*n*-C9H19
*n*-C15H31

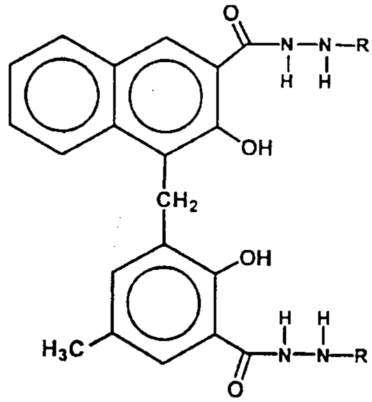

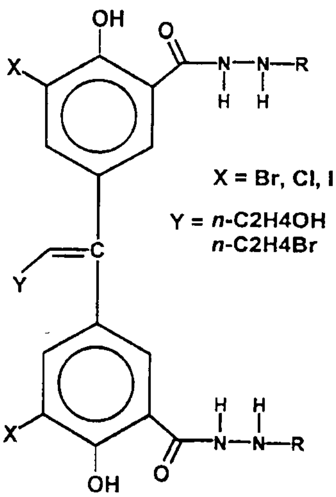

X = Br, Cl, I
Y = *n*-C2H4OH
*n*-C2H4Br

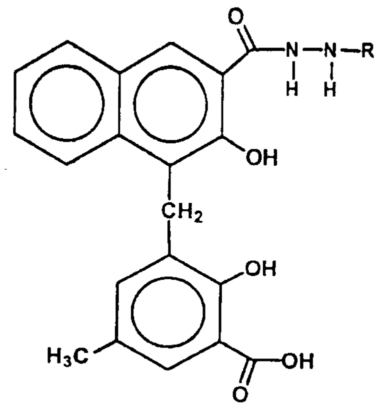

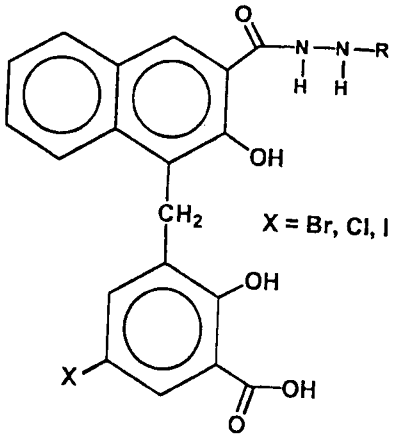

X = Br, Cl, I

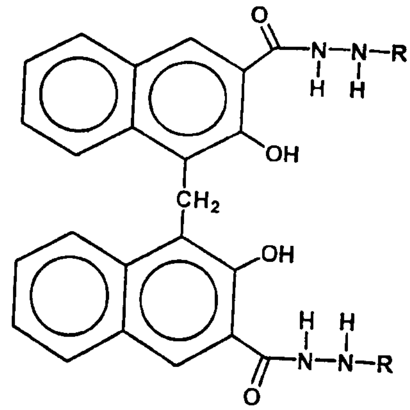

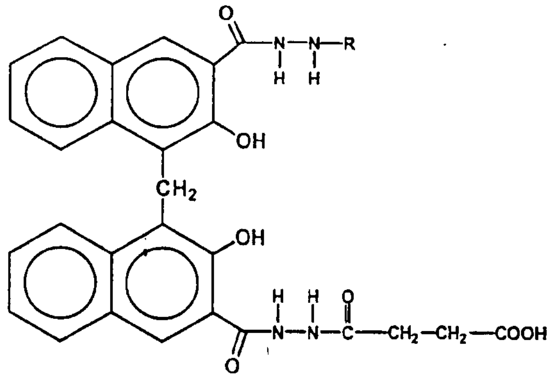

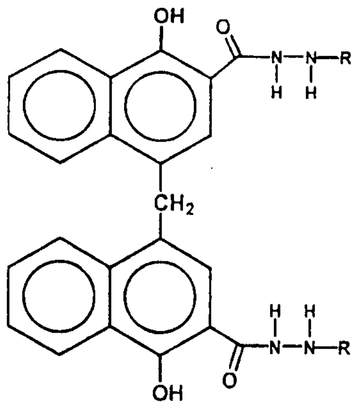

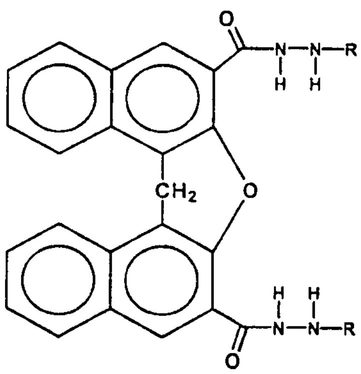

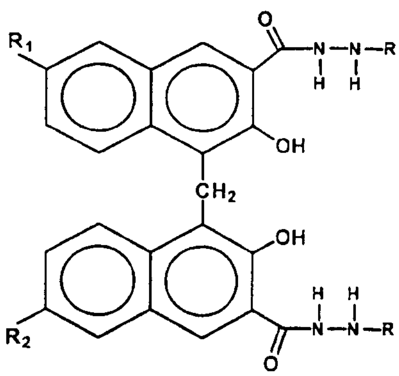

$R_1=R_2=MeO$
$R_1,R_2=MeO,H$
$R_1=R_2=Me$
$R_1,R_2=Me,H$
$R_1,R_2=Me,MeO$
$R_1=R_2=NH_2$
$R_1,R_2=Me,NH_2$
$R_1,R_2=H,NH_2$
$R_1,R_2=MeO,NH$

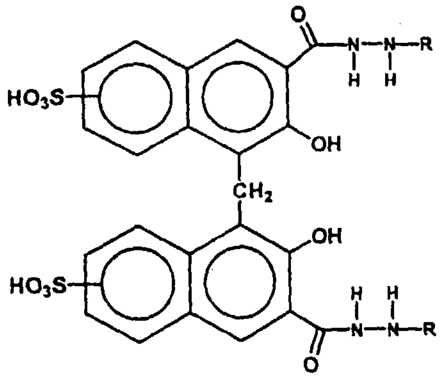

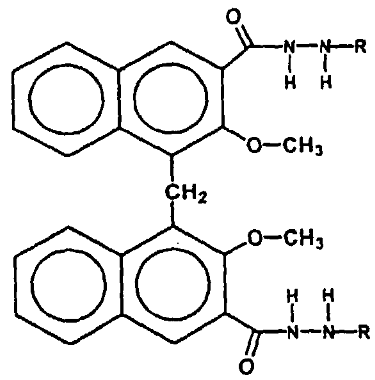

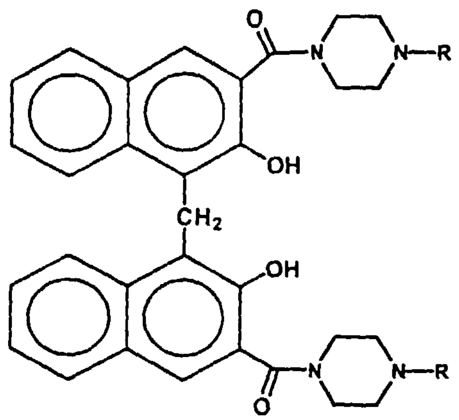

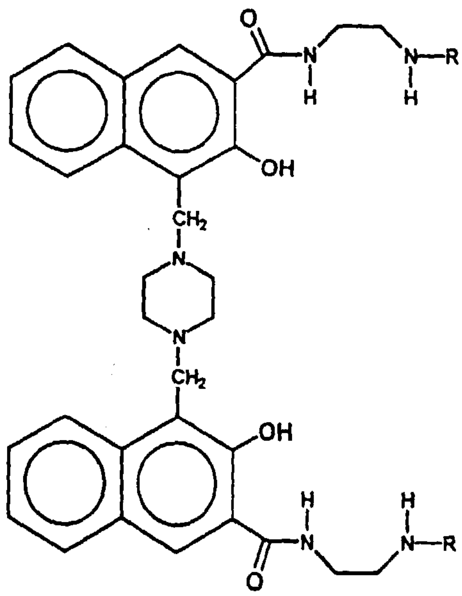

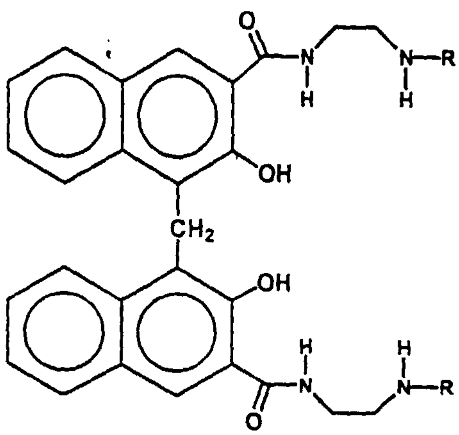

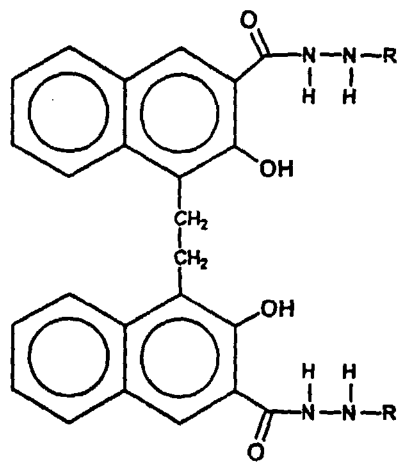

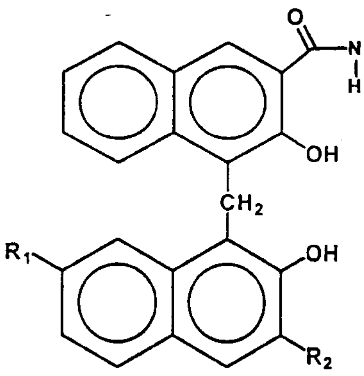

R1, R2 = H, H
R1, R2 = MeO, H
R1, R2 = H, Me

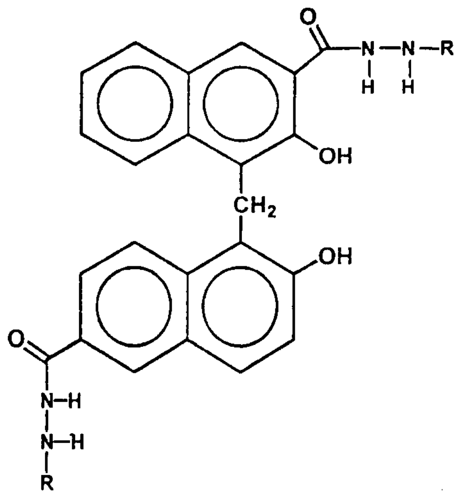

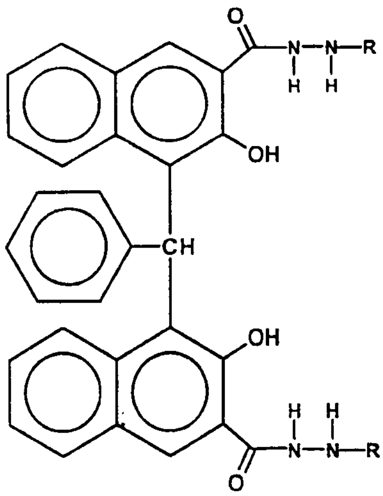

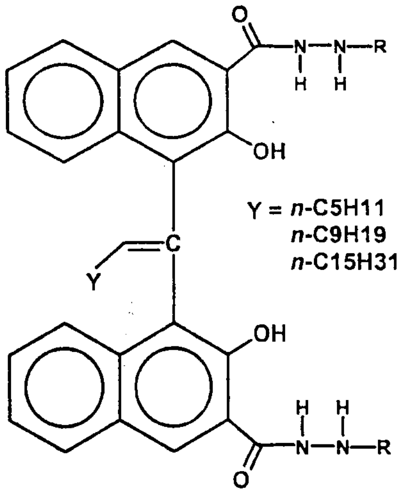

Y = *n*-C5H11
*n*-C9H19
*n*-C15H31

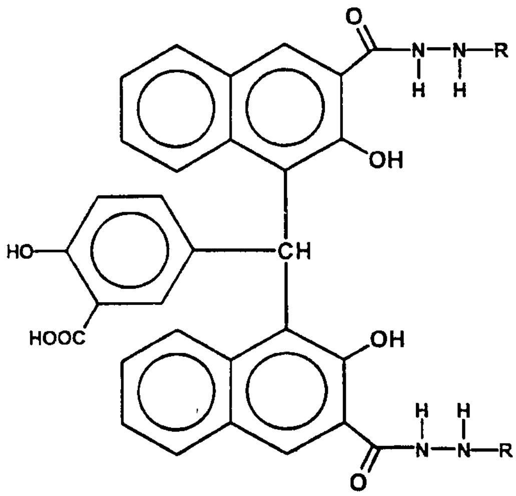

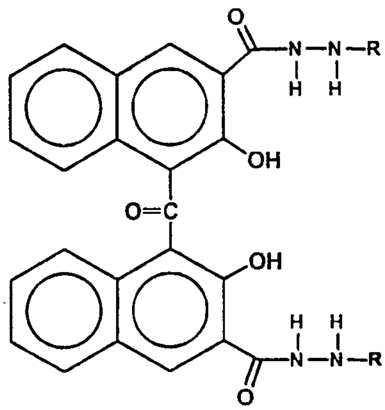

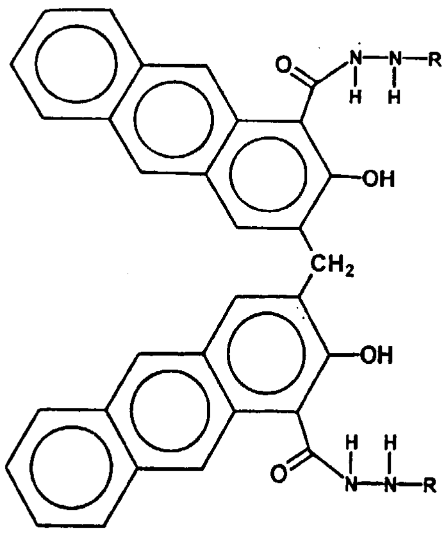

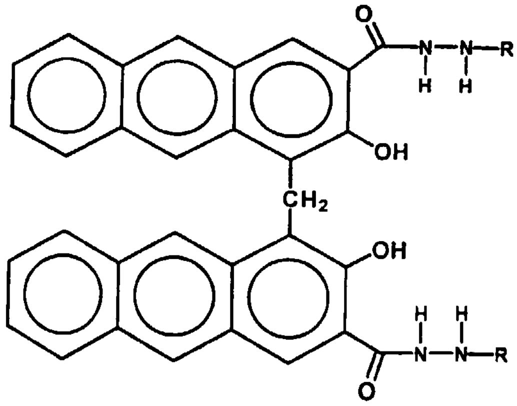

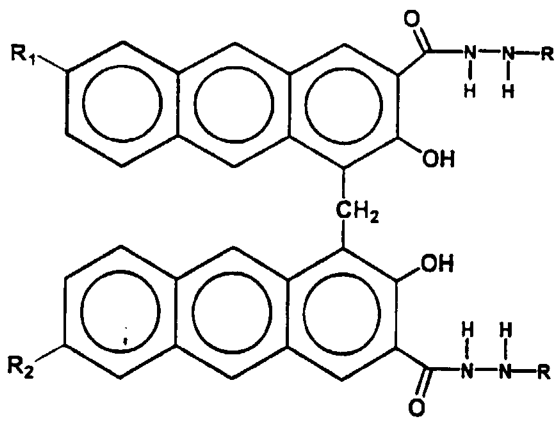

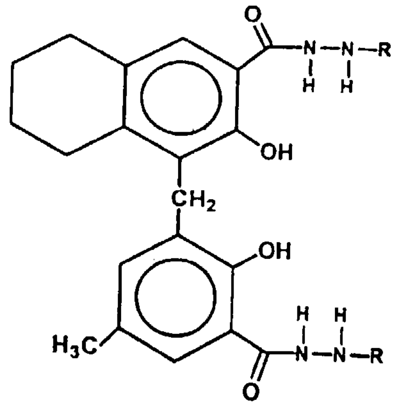

$R_1=R_2=MeO$    $R_1=R_2=Me$    $R_1=R_2=NH_2$

$R_1,R_2=MeO,H$    $R_1,R_2=Me,H$    $R_1,R_2=H,NH_2$

$R_1,R_2=MeO,NH_2$    $R_1,R_2=Me,NH_2$    $R_1,R_2=Me,MeO$

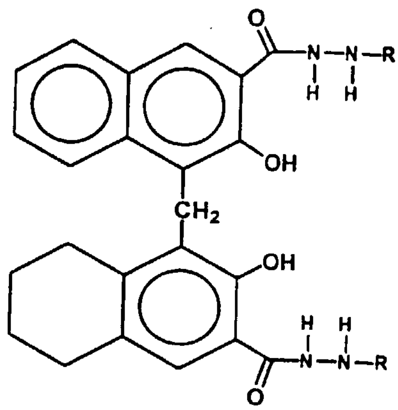

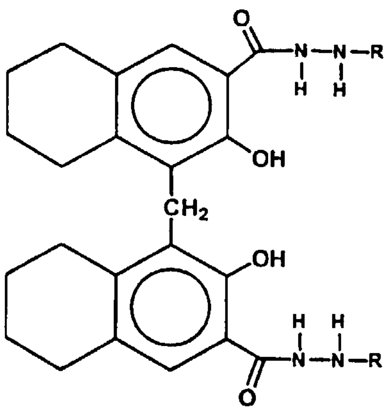

**21.** Compound according to any of the previous claims for use as a medicament and/or diagnosticum.

**22.** Compound according to any of the previous claims for localization and identification of necrotic tissues and/or components thereof.

**23.** Use of a compound according to any of the claims 1-20 for preparing a medicament and/or a diagnosticum.

**24.** Use of a compound according to any of the claims 1-20 for manufacturing a diagnosticum for identifying and for localizing necrotic tissues and/or components thereof.

**25.** Use of a compound according to any of the claims 1-20 for manufacturing a blood pool agent for MRI and/or radiography angiography, especially coronary angiography, and for manufacturing a diagnosticum and/or medicament for identifying and/or treating malfunctions of the renal and/or hepatobiliary system.

**26.** Process for providing a compound according to any of the claims 1-20 comprising the steps of reacting together two or more of the targeting agents, labelling agents, linking agents and spacing agents, or precursors thereof, under reactive conditions.

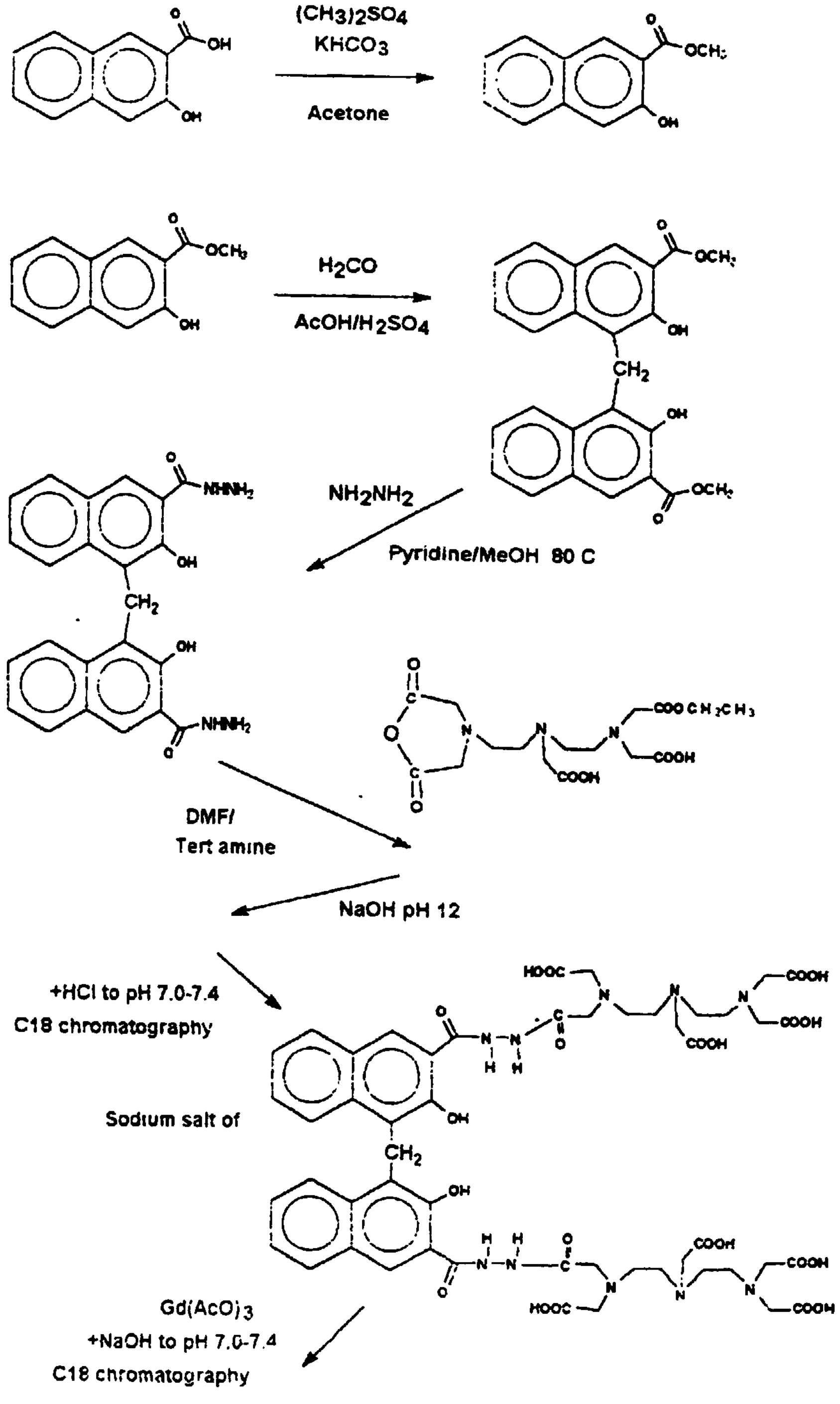


Bis Gd-complex of title compound

Fig. 1

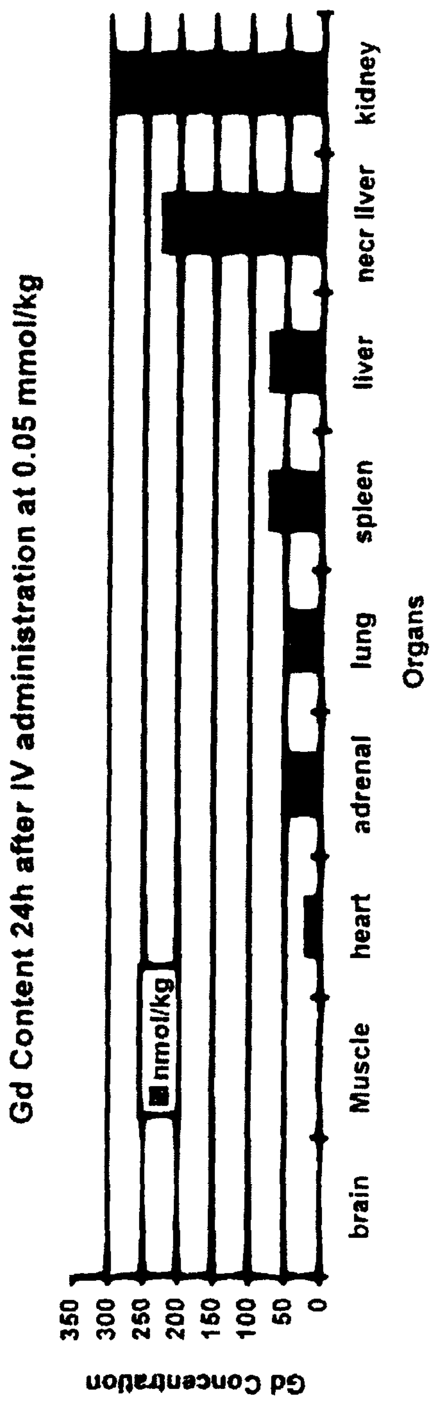
Gd Content 24h after IV administration at 0.05 mmol/kg
nmol/kg
Gd Concentration
350
300
250
200
150
100
50
0
brain
Muscle
heart
adrenal
lung
spleen
liver
necr liver
kidney
Organs

Fig. 2

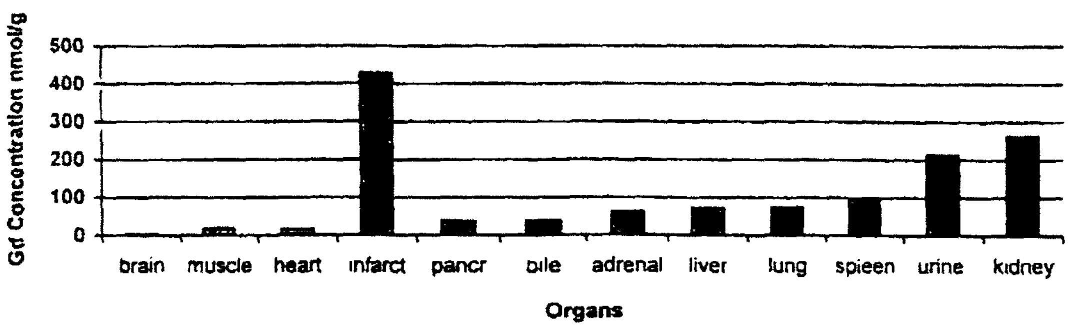
Gd Concentration nmol/g
500
400
300
200
100
0
brain
muscle
heart
infarct
pancr
bile
adrenal
liver
lung
spleen
urine
kidney
Organs

Fig. 3

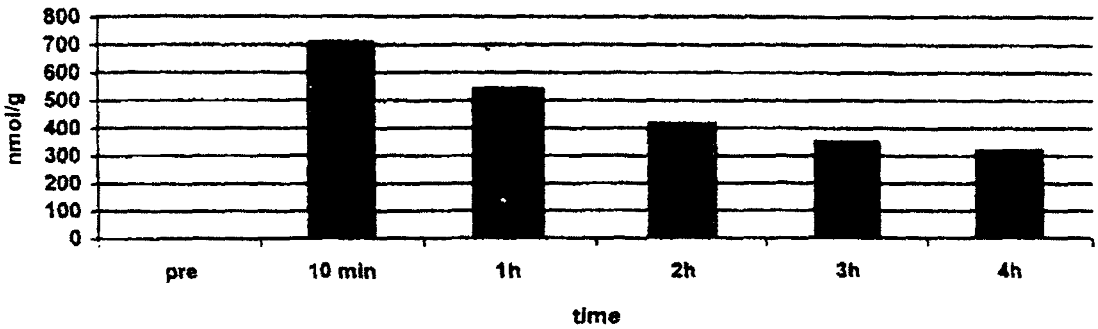
nmol/g
800
700
600
500
400
300
200
100
0
pre
10 min
1h
2h
3h
4h
time

Fig. 4

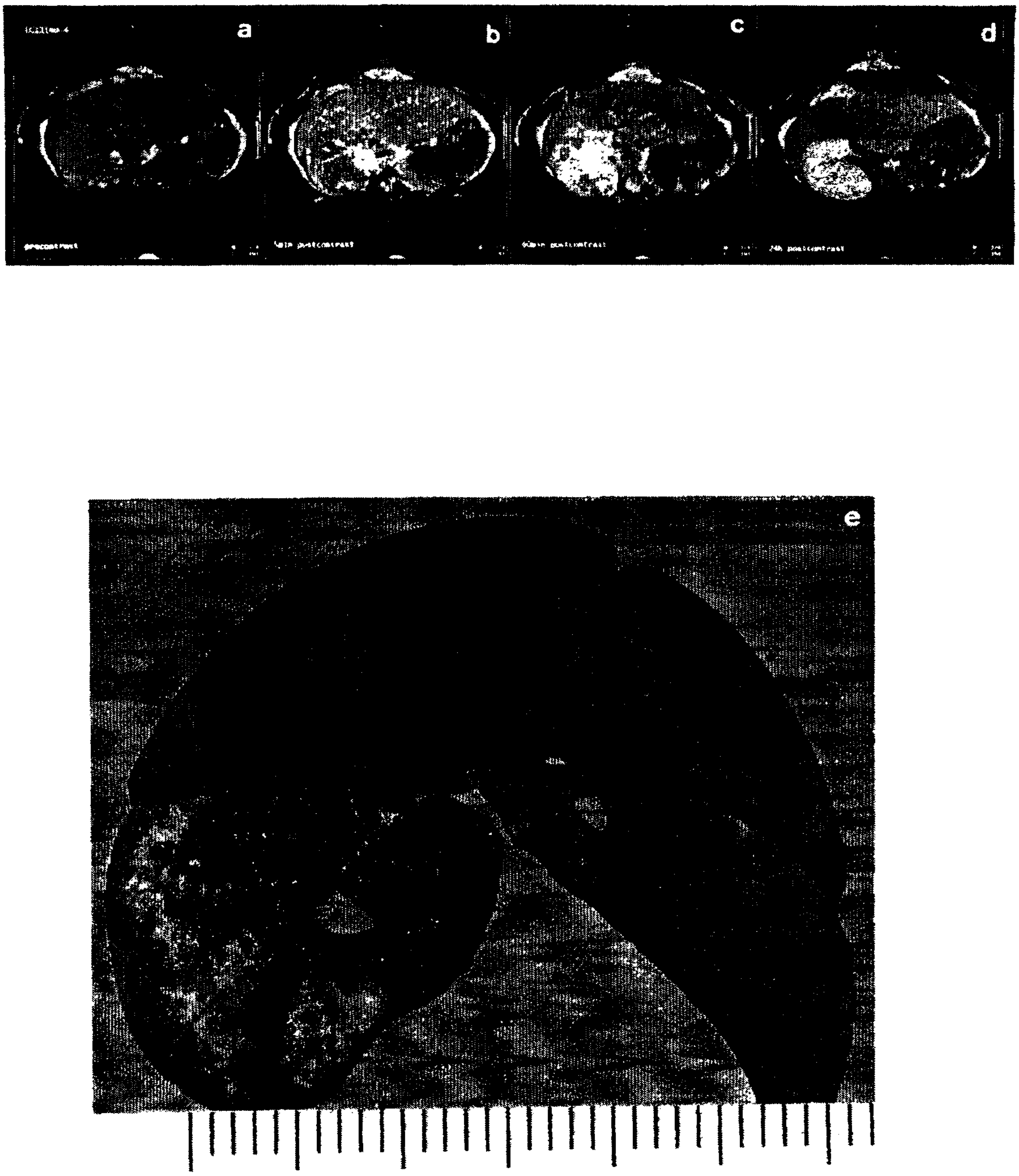


Fig. 5

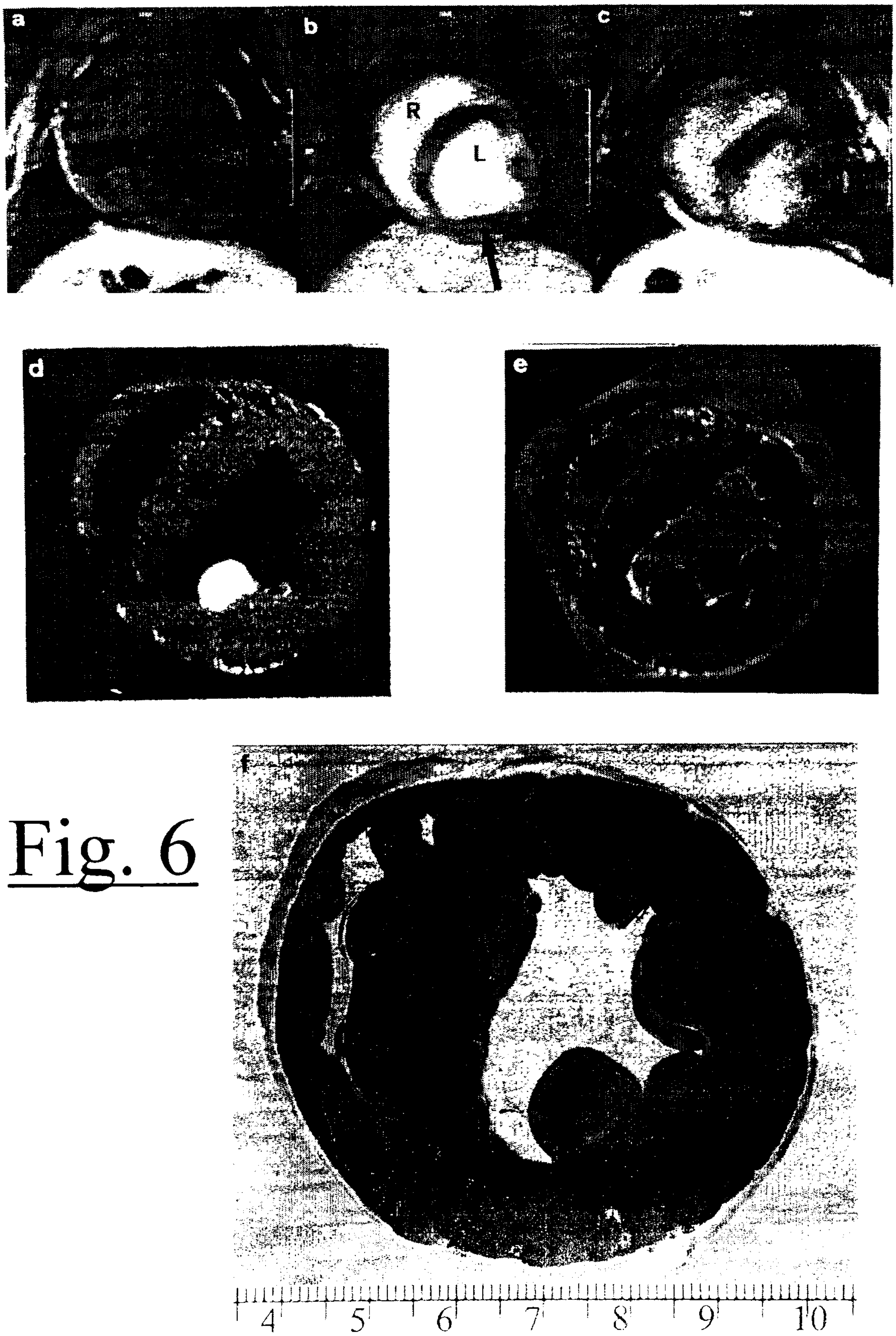


Fig. 6

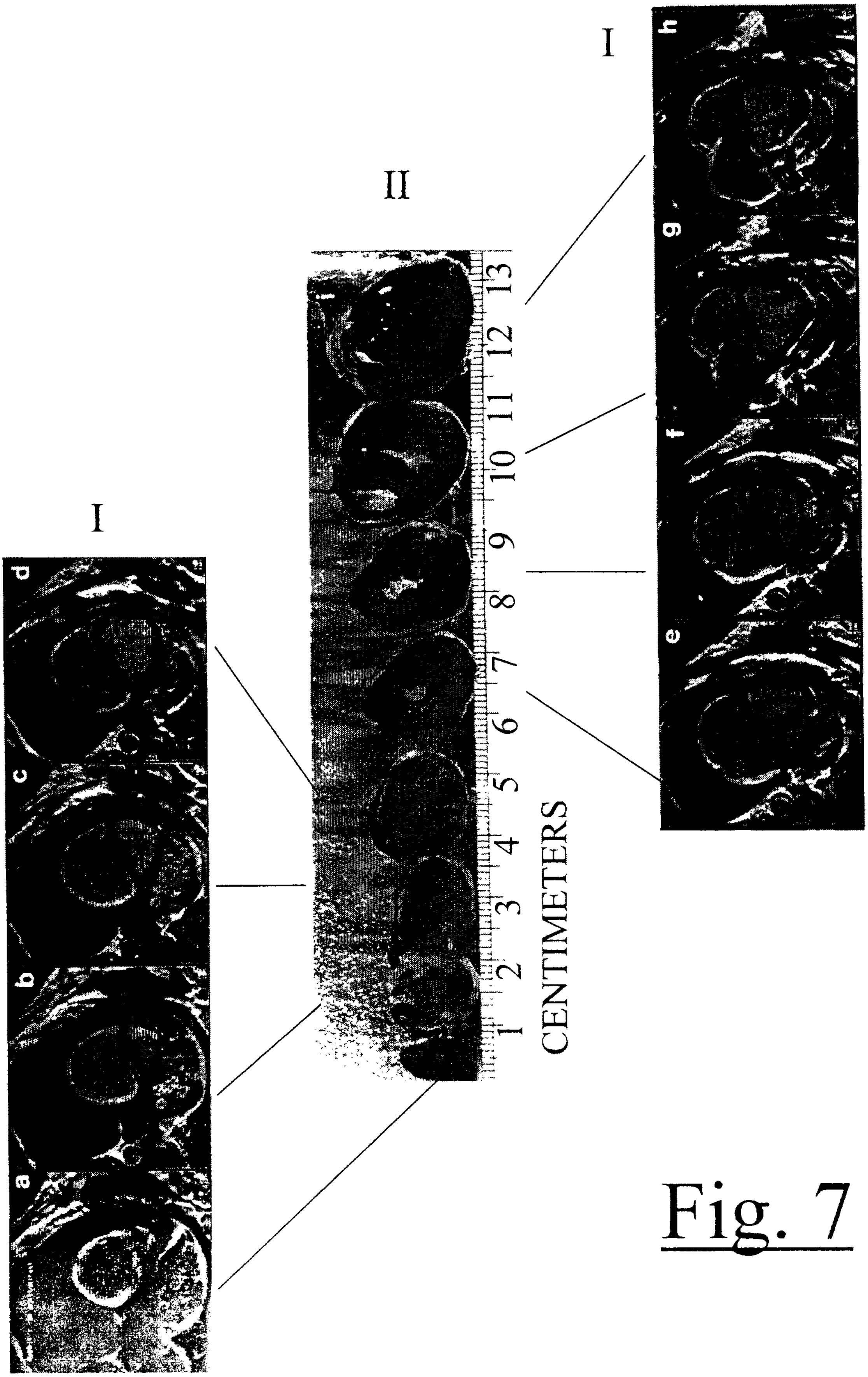


Fig. 7

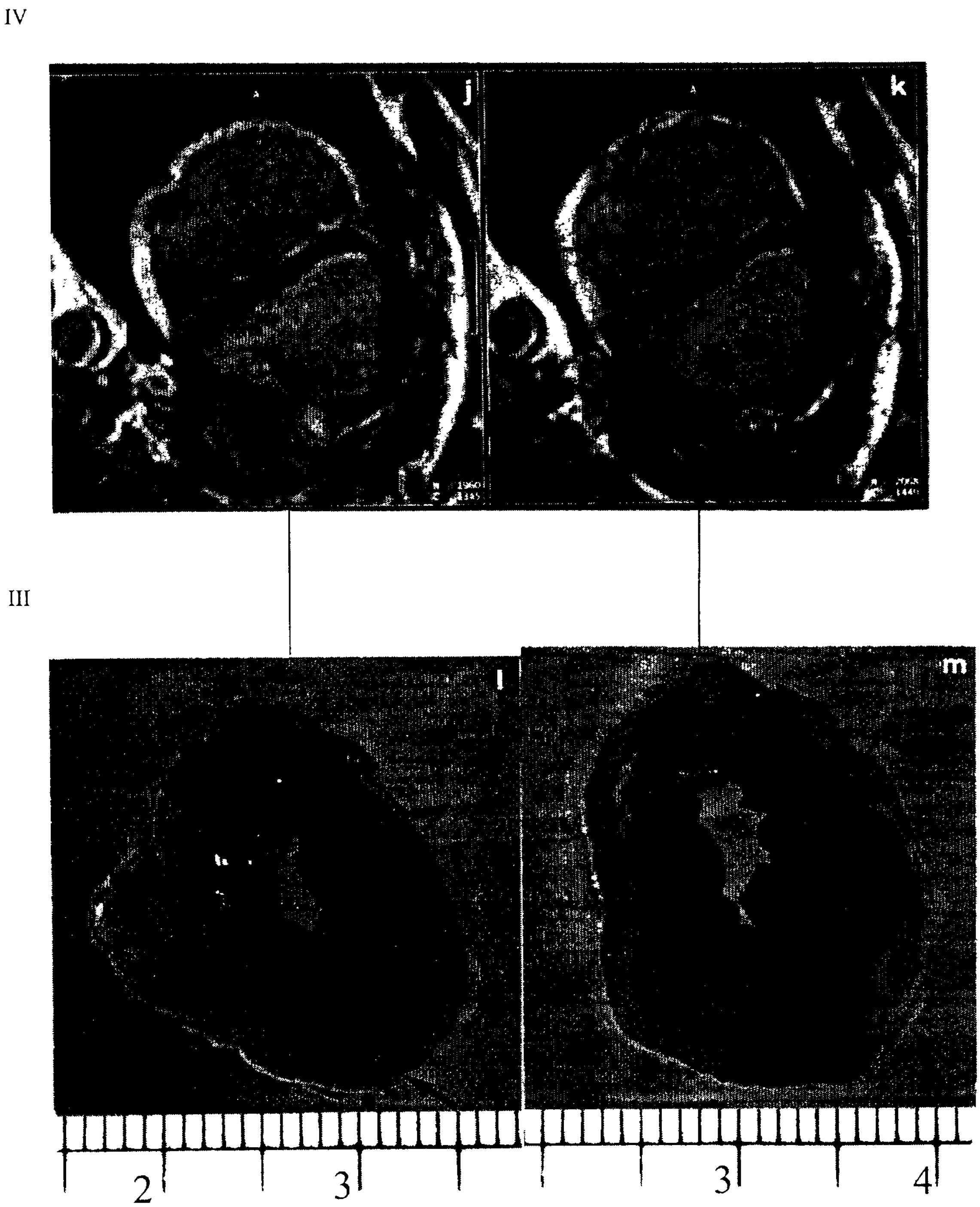


Fig. 7

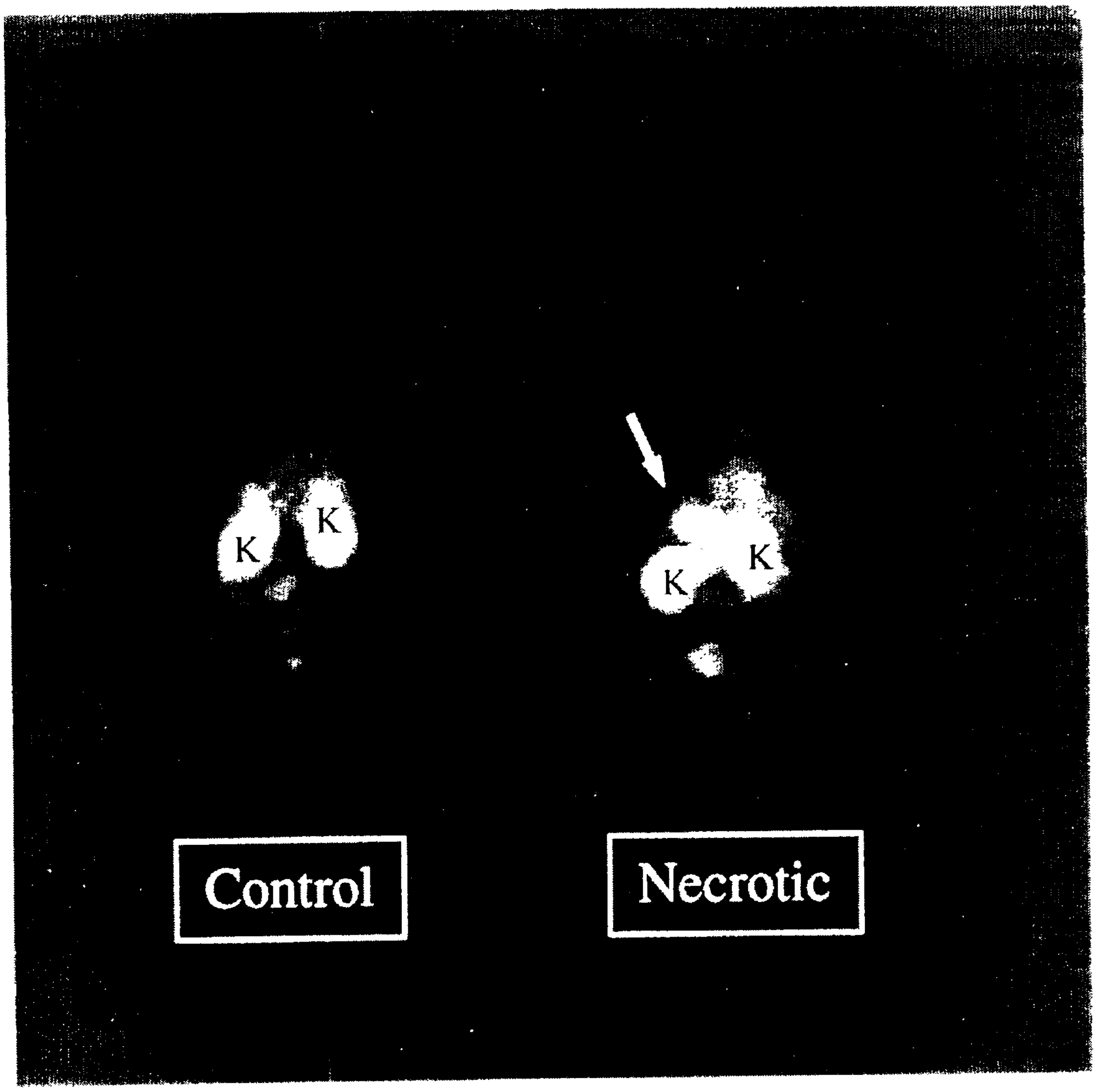


Fig. 8

European Patent Office

# PARTIAL EUROPEAN SEARCH REPORT

which under Rule 45 of the European Patent Convention shall be considered, for the purposes of subsequent proceedings, as the European search report

Application Number
EP 99 20 0327

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WO 98 06386 A (BOEHRINGER MANNHEIM GMBH ;DICKHAUT JOACHIM (DE)) 19 February 1998 (1998-02-19) | 1 | A61K49/00<br>A61K47/48 |
| Y | * claims * | 1-26 | |
| | --- | | |
| Y | WO 95 27705 A (BRACCO INT BV) 19 October 1995 (1995-10-19) * claims * | 1-26 | |
| | --- | | |
| Y | US 5 476 644 A (ILLIG CARL R ET AL) 19 December 1995 (1995-12-19) * column 4, line 45 - line 46; claims * | 1-26 | |
| | ----- | | |

TECHNICAL FIELDS SEARCHED (Int.Cl.7)

A61K

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| THE HAGUE | 8 November 1999 | Berte, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

European Patent Office

## INCOMPLETE SEARCH SHEET C

Application Number
EP 99 20 0327

Claim(s) searched incompletely:
1-26

Reason for the limitation of the search:

Present claims 1-26 relate to an extremely large number of possible compounds
. Support within the meaning of Article 84 EPC and/or disclosure within the meaning of Article 83 EPC is to be found, however, for only a very small proportion of the compounds claimed. In the present case, the claims so lack support, and the application so lacks disclosure, that a meaningful search over the whole of the claimed scope is impossible. Consequently, the search has been carried out for those parts of the claims which appear to be supported and disclosed, namely those parts relating to the compounds.
The search has been performed for those compounds prepared in the examples and closely related homologous compounds .